# EUROPEAN PATENT APPLICATION

(11) **EP 4 744 607 A2**
(43) Date of publication of application: **20.05.2026**
(21) Application number: 25216735.8
(22) Date of filing: 18.11.2025
(51) Int. Cl.: A61B 18/12, A61M 13/00

(54) **AIR MANAGEMENT IN SURGICAL SMOKE EVALUATION SYSTEMS**

(30) Priority: 18.11.2024 US 202418950899
(71) Applicant: Cilag GmbH International, 6300 Zug (CH)
(72) Inventor: ASHER, Ryan M., Cincinnati, 45242 (US); HERMAN, Daniel C., Cincinnati, 45242 (US); HORNER, Shawn K., Cincinnati, 45242 (US); ALDRIDGE, Jeffrey, Cincinnati, 45242 (US); SCHULTE, John B., Cincinnati, 45242 (US); MINNELLI, Patrick J., Cincinnati, 45242 (US); VOEGELE, Aaron C., Cincinnati, 45242 (US); MORGAN, Joshua, Cincinnati, 45242 (US); SWENSON, Rachael A., Cincinnati, 45242 (US); WEED, III, John A., Cincinnati, 45242 (US)
(74) Representative: Carpmaels & Ransford LLP

(57) **Abstract**

A surgical system is disclosed including a power source operable to provide a source amount of power, a first surgical module electrically coupled to the power source, a second surgical module electrically coupled to the power source, and a controller operable to determine a free amount of power available to the second surgical module from the power source. The first surgical module is transitionable between an idle state, in which the first surgical module draws a first amount of power from the power source, and an active state, in which the first surgical module draws a second amount of power greater than the first amount of power from the power source. The free amount of power corresponds to a difference between the source amount of power and an amount of power being drawn by the first surgical module.

## Description

### BACKGROUND

The present disclosure relates to surgical systems and, more particularly, insufflation and smoke evacuation systems used in surgical systems.

During a surgical procedure involving an energy device, insufflators are often used to provide insufflation to a patient's body cavity and thereby enhance visibility and access to the same. During the surgical procedure, smoke can be generated at a surgical site as the energy device is utilized. Surgical smoke evacuators are configured to evacuate smoke, as well as fluid and/or particulate, from the surgical site.

### BRIEF DESCRIPTION OF THE DRAWINGS

The following figures are included to illustrate certain aspects of the present disclosure, and should not be viewed as exclusive embodiments. The subject matter disclosed is capable of considerable modifications, alterations, combinations, and equivalents in form and function, without departing from the scope of this disclosure.
FIG. 1 is a block diagram of a computer-implemented interactive surgical system, in accordance with at least one aspect of the present disclosure.
FIG. 2 is a diagram of various modules, including an energy module, an evacuation module, and an insufflation module, and other components that are combinable to customize modular surgical systems, in accordance with at least one aspect of the present disclosure.
FIG. 3 is the energy module of FIG. 2 and various surgical instruments usable therewith, in accordance with at least one aspect of the present disclosure.
FIG. 4A is a first illustrative modular surgical system configuration including a header module and a display screen that renders a graphical user interface (GUI) for relaying information regarding modules connected to the header module, in accordance with at least one aspect of the present disclosure.
FIG. 4B is an isometric view of the modular surgical system shown in FIG. 4A mounted to a cart, in accordance with at least one aspect of the present disclosure.
FIG. 5 is a second illustrative modular surgical system configuration including a header module, a display screen, two energy modules, and an evacuation module connected together and mounted to a cart, in accordance with at least one aspect of the present disclosure.
FIG. 6 is a block diagram of an example modular surgical system, in accordance with at least one aspect of the present disclosure.
FIG. 7 is a schematic depicting internal components of the insufflation module of FIG. 2, in accordance with at least one aspect of the present disclosure.
FIG. 8 is a schematic depicting internal components within the evacuation module of FIG. 2, in accordance with at least one aspect of the present disclosure.
FIG. 9 is a block diagram of a modular surgical system, in accordance with at least one aspect of the present disclosure.
FIG. 10 is a block diagram of a modular surgical system, in accordance with at least one aspect of the present disclosure.
FIG. 11 is a flow diagram for controlling the modular surgical system of FIG. 11, in accordance with at least one aspect of the present disclosure.
FIG. 12 is a block diagram of a modular surgical system, in accordance with at least one aspect of the present disclosure.
FIG. 13 is a trocar including a luer lock connection, in accordance with at least one aspect of the present disclosure.
FIG. 14 illustrates a block diagram of a modular surgical system including an insufflation module providing insufflation gas to a patient's body cavity, an evacuation module drawing gas from the patient's body cavity, a common pump, three three-way valves, and three trocars in accordance with at least one aspect of the present disclosure.
FIG. 15 illustrates the modular surgical system of FIG. 14 in an insufflation state in which the common pump provides additional insufflation to the patient's body cavity, in accordance with at least one aspect of the present disclosure.
FIG. 16 illustrates the modular surgical system of FIG. 14 in an evacuation state in which the common pump draws additional smoke from the patient's body cavity, in accordance with at least one aspect of the present disclosure.
FIG. 17 illustrates the modular surgical system of FIG. 14 in a cleaning state, in accordance with at least one aspect of the present disclosure.
FIG. 18 illustrates the modular surgical system of FIG. 14 further including a tertiary filter, in accordance with at least one aspect of the present disclosure.
FIG. 19 illustrates a block diagram of another modular surgical system including an insufflation module providing insufflation gas to a patient's body cavity, an evacuation module drawing gas from the patient's body cavity, a common pump, three three-way valves, a four-way valve, and three-trocars, in accordance with at least one aspect of the present disclosure.
FIG. 20 illustrates the modular surgical system of FIG. 19 in an insufflation state in which the common pump provides additional insufflation to the patient's body cavity, in accordance with at least one aspect of the present disclosure.
FIG. 21 illustrates the modular surgical system of FIG. 19 in an evacuation state in which the common pump draws additional smoke from the patient's body cavity, in accordance with at least one aspect of the present disclosure.
FIG. 22 illustrates a block diagram of another modular surgical system including an insufflation module providing insufflation gas to a patient's body cavity, an evacuation module drawing gas from the patient's body cavity, a common pump, three three-way valves, and four trocars, in accordance with at least one aspect of the present disclosure.
FIG. 23 illustrates a block diagram of a modular surgical system, in accordance with at least one aspect of the present disclosure.
FIG. 24 illustrates flow diagrams for controlling the modular surgical system of FIG. 23, in accordance with at least one aspect of the present disclosure.

### DETAILED DESCRIPTION

Applicant of the present application owns the following U.S. Patent Applications filed concurrently herewith, the disclosure of each of which is hereby incorporated by reference in their entirety herein:
U.S. Patent Application No. 18/950,801, titled IMPROVED FILTER LIFE IN SURGICAL smoke EVACUATION SYSTEMS;
U.S. Patent Application No. 18/951,268, titled INTELLIGENT INSUFFLATION AND SMOKE EVACUATION; and
U.S. Patent Application No. 18/951,342, titled SETTING EVACUATION MOTOR SPEEDS FOR SURGICAL TOOL EVACUATION MODULES.

The present disclosure relates to energy devices, insufflation systems for providing insufflation gas to a patient, and evacuation systems for evacuating smoke and/or other fluids and/or particulates from a surgical site.

Smoke is often generated during a surgical procedure that utilizes one or more energy devices. Energy devices use energy to affect (treat) tissue. In an energy device, the energy is supplied by a generator. Energy devices include devices with tissue-contacting electrodes, such as an electrosurgical device having one or more radio frequency (RF) electrodes, and devices with vibrating surfaces, such as an ultrasonic device having an ultrasonic blade. For an electrosurgical device, a generator is configured to generate oscillating electric currents to energize the electrodes. For an ultrasonic device, a generator is configured to generate ultrasonic vibrations to energize the ultrasonic blade. Generators are further described herein. An evacuation module is utilized to control an amount of smoke generated by the energy devices during use thereof.

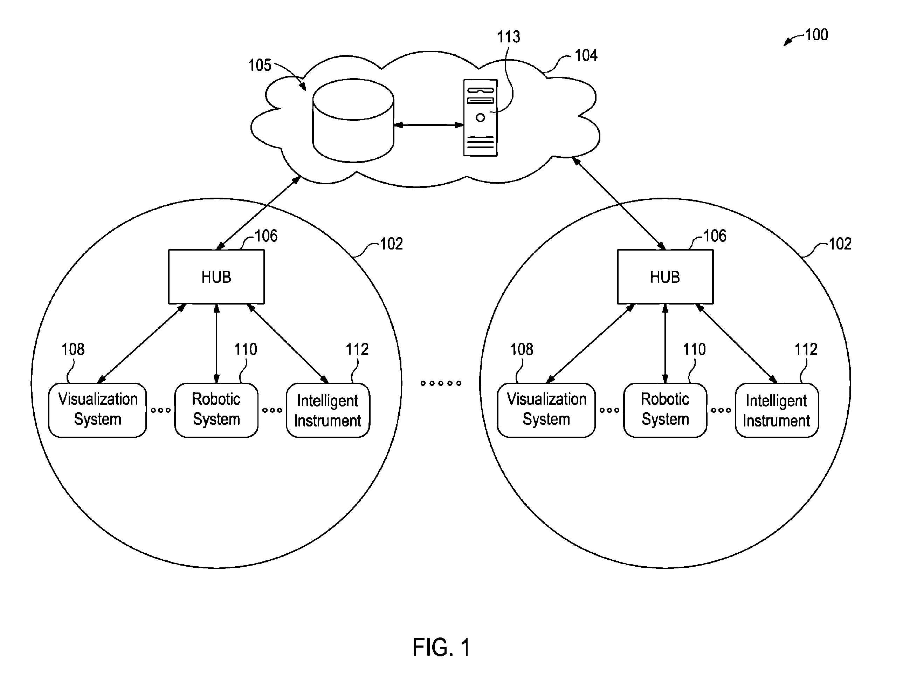

FIG. 1 is a block diagram of a computer-implemented interactive surgical system 100 (hereinafter "the surgical system 100") that may be used in accordance with at least one aspect of the present disclosure. The surgical system 100 includes one or more sub-surgical systems 102 and a cloud-based system (e.g., the cloud 104) that may include a remote server 113 in communication with a storage device 105. Each sub-surgical system 102 includes at least one surgical hub 106 in communication with the cloud 104 that may include a remote server 113.

In one example, as illustrated in FIG. 1, each sub-surgical system 102 includes a visualization system 108, a robotic system 110, and a handheld intelligent surgical instrument 112, which are configured to communicate with one another and/or the hub 106. In some aspects, each sub-surgical system 102 may include an M number of hubs 106, an N number of visualization systems 108, an O number of robotic systems 110, and a P number of handheld intelligent surgical instruments 112, where M, N, O, and P are integers greater than or equal to one. The surgical system 100 is described in more detail in U.S. Patent No. 11,666,368, entitled "METHOD FOR CONSTRUCTING AND USING A MODULAR SURGICAL ENERGY SYSTEM WITH MULTIPLE DEVICES", issued June 6, 2023, and is hereby incorporated by reference in its entirety herein.

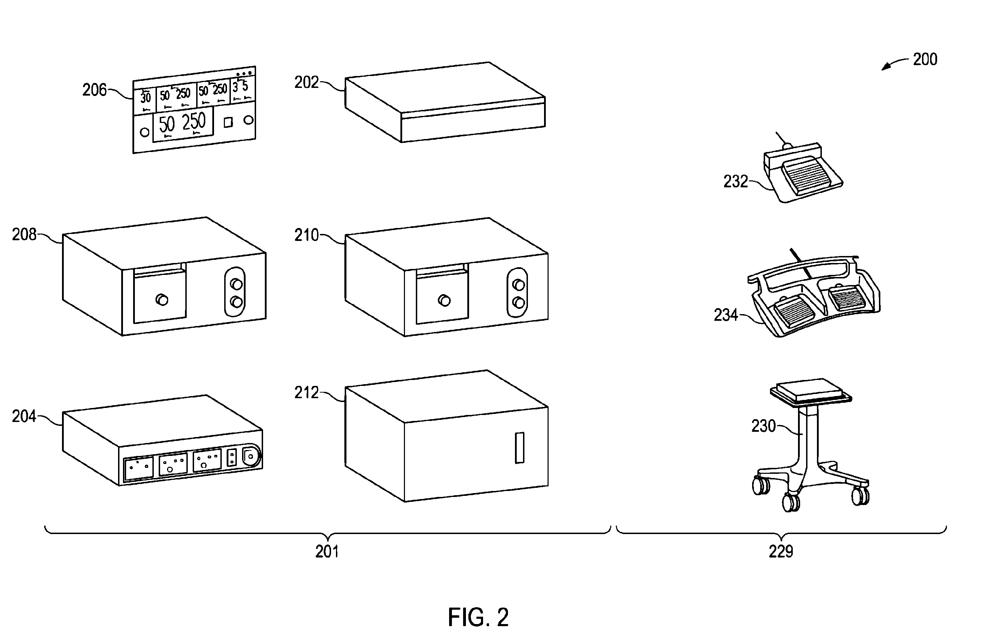

Referring now to FIG. 2, an example surgical hub 106 (FIG. 1) can be embodied as a modular surgical system 200 that can include a variety of different modules 201 that are connectable together in a stacked configuration. In one aspect, the modules 201 can be both physically and communicably coupled together when stacked or otherwise connected together into a singular assembly. Further, the modules 201 can be interchangeably connectable together in different combinations or arrangements. In one aspect, each of the modules 201 can include a consistent or universal array of connectors disposed along their upper and lower surfaces, thereby allowing any module 201 to be connected to another module 201 in any arrangement (except that, in some aspects, a particular module type, such as the header module 202, can be configured to serve as the uppermost module within the stack, for example). In an alternative aspect, the modular surgical system 200 can include a housing that is configured to receive and retain the modules 201. The modular surgical system 200 can also include a variety of different components or accessories that are also connectable to or otherwise associatable with the modules 201.

The modular surgical system 200 can be assembled from a variety of different modules 201, some examples of which are illustrated in FIG. 2. Each of the different types of modules 201 can provide different functionality, thereby allowing the modular surgical system 200 to be assembled into different configurations to customize the functions and capabilities of the modular surgical system 200 (e.g., by customizing the modules 201 that are included in each modular surgical system 200). The modules 201 of the modular surgical system 200 can include, for example, a header module 202 (which can include a display screen 206), an energy module 204, an evacuation module 208, an insufflation module 210, and a visualization module 212.

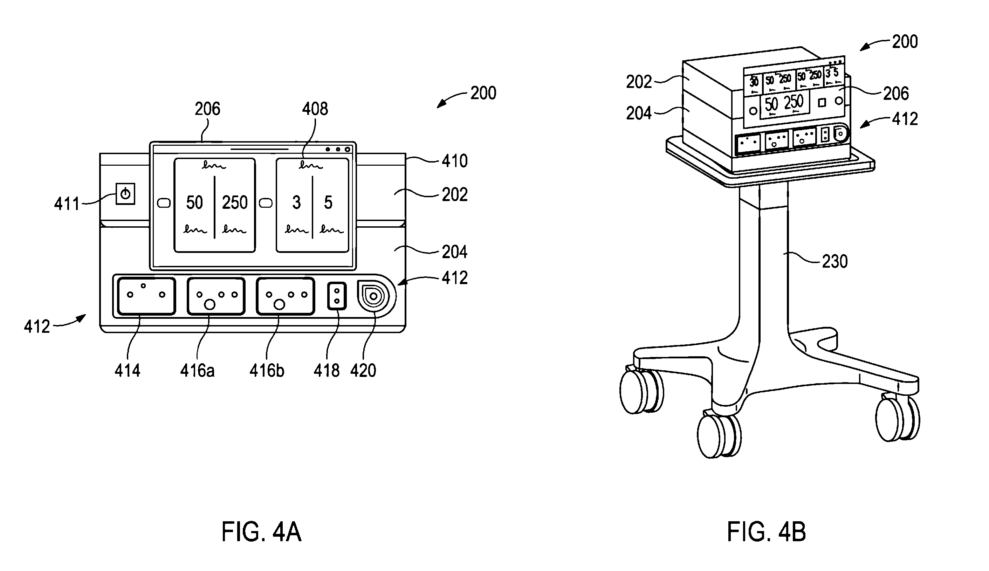

In the depicted aspect, the header module 202 is configured to serve as the top or uppermost module within the modular surgical system stack and can thus lack connectors along its top surface. In another aspect, the header module 202 can be configured to be positioned at the bottom or the lowermost module within the modular surgical system stack (i.e., a "footer" module) and can thus lack connectors along its bottom surface. In yet another aspect, the header module 202 can be configured to be positioned at an intermediate position within the modular surgical system stack and can thus include connectors along both its bottom and top surfaces. The header module 202 can be configured to control the system-wide settings of each module 201 and component connected thereto through physical controls 411 (FIG. 4A) thereon and/or a graphical user interface (GUI) 408 (FIG. 4A) rendered on the display screen 206. Such settings could include the activation of the modular surgical system 200, the volume of alerts, the footswitch settings, the settings icons, the appearance or configuration of the user interface, the surgeon profile logged into the modular surgical system 200, and/or the type of surgical procedure being performed. The header module 202 can also be configured to provide communications, processing, and/or power for the modules 201 that are connected to the header module 202.

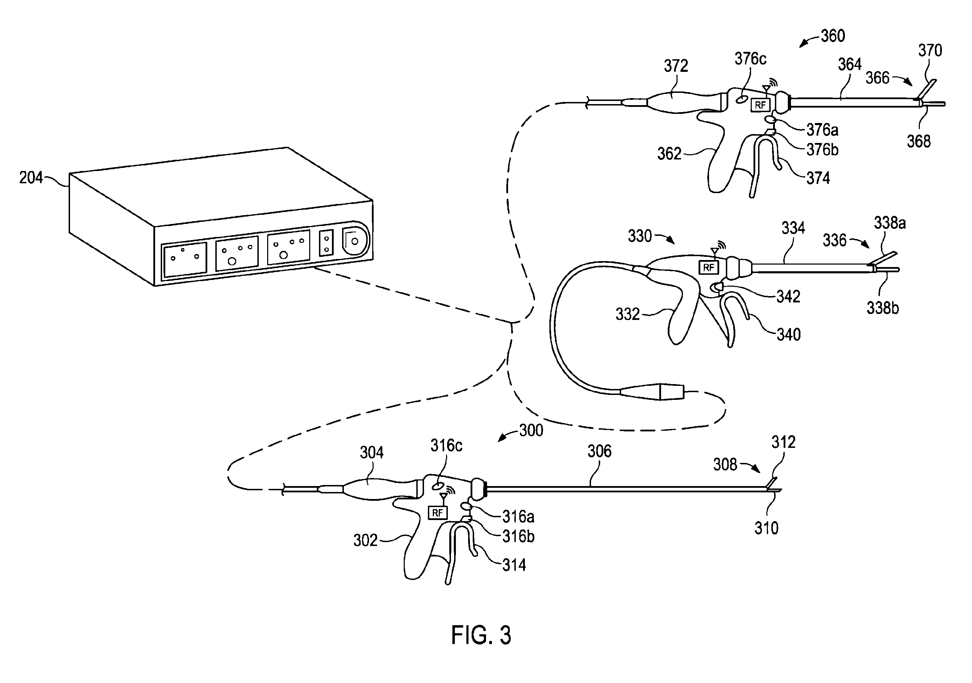

The energy module 204, alternately referred to as a generator module, can be configured to generate one or multiple energy modalities for driving electrosurgical and/or ultrasonic surgical instruments connected thereto. For example, referring to FIG. 3, the generator 204 is configured to drive multiple surgical instruments 300, 330, 360. The first surgical instrument is an ultrasonic surgical instrument 300 and comprises a handpiece 302 (HP), an ultrasonic transducer 304, a shaft 306, and an end effector 308. The end effector 308 comprises an ultrasonic blade 310 acoustically coupled to the ultrasonic transducer 304 and a clamp arm 312. The handpiece 302 comprises a trigger 314 to operate the clamp arm 312 and a combination of toggle buttons 316a, 316b, 316c to energize and drive the ultrasonic blade 310 or other function. The toggle buttons 316a-c can be configured to energize the ultrasonic transducer 304 with the generator 204.

The generator 204 is also configured to drive the second surgical instrument 330, which is an RF electrosurgical instrument and comprises a handpiece 332 (HP), a shaft 334, and an end effector 336. The end effector 336 comprises electrodes in clamp arms 338a, 338b and return through an electrical conductor portion of the shaft 334. The electrodes are coupled to and energized by a bipolar energy source within the generator 204. The handpiece 332 includes a trigger 340 manually actuatable to operate the clamp arms 338a,b, and an energy button 342 to actuate an energy switch to energize the electrodes in the end effector 336.

The generator 204 is also configured to drive the third surgical instrument 360, which is a multifunction surgical instrument 360 and comprises a handpiece 362 (HP), a shaft 364, and an end effector 366. The end effector 366 comprises an ultrasonic blade 368 and a clamp arm 370. The ultrasonic blade 368 is acoustically coupled to an ultrasonic transducer 372. The handpiece 362 includes a trigger 374 to operate the clamp arm 370, and a combination of toggle buttons 376a, 376b, 376c to energize and drive the ultrasonic blade 368 or other function. The toggle buttons 376a-c can be configured to energize the ultrasonic transducer 372 with the generator 204 and energize the ultrasonic blade 368 with a bipolar energy source also contained within the generator 204. Further aspects of the surgical instruments are described in U.S. Patent No. 10,624,691, entitled "TECHNIQUES FOR OPERATING GENERATOR FOR DIGITALLY GENERATING ELECTRICAL SIGNAL WAVEFORMS AND SURGICAL INSTRUMENTS", issued April 21, 2020, which is herein incorporated by reference in its entirety herein.

The evacuation module 208 (FIG. 2) can be configured to evacuate smoke, fluid, and/or particulates generated by the application of therapeutic energy to the tissue by one or more of the surgical instruments 300, 330, 360. Example evacuation modules are described in more detail elsewhere herein, as well as in U.S. Patent No. 11,602,393, entitled "SURGICAL EVACUATION SENSING AND GENERATOR CONTROL", issued March 14, 2023, which is hereby incorporated by reference in its entirety herein. The insufflation module 210 (FIG. 2) can be configured to blow air or gas into a body cavity of a patient to inflate it for diagnostic or surgical procedures, thereby providing better visibility and access during procedures.

The visualization module 212 (FIG. 2) can be configured to interface with visualization devices (i.e., scopes) and accordingly provide increased visualization capabilities. Example visualization modules and systems are described in more detail in U.S. Patent No. 11,284,963, entitled "METHOD OF USING IMAGING DEVICES IN SURGERY", issued March 29, 2022, which is hereby incorporated by reference in its entirety herein.

Referring again to FIG. 2, the modular surgical system 200 can further include a variety of accessories 229 that are connectable to the modules 201 for controlling the functions thereof or that are otherwise configured to work in conjunction with the modular surgical system 200. The accessories 229 can include, for example, a single-pedal footswitch 232, a dual-pedal footswitch 234, and a cart 230 for supporting the modular surgical system 200 thereon. The footswitches 232, 234 can be configured to control the activation or function of particular energy modalities output by the energy module 204, for example.

By utilizing modular components, the depicted modular surgical system 200 provides a surgical platform that grows with the availability of technology and is customizable to the needs of the facility and/or surgeons. Further, the modular surgical system 200 supports combo devices (e.g., dual electrosurgical and ultrasonic energy generators) and supports software-driven algorithms for customized tissue effects. Still further, the surgical system architecture reduces the capital footprint by combining multiple technologies critical for surgery into a single system.

The various modular components utilizable in connection with the modular surgical system 200 can include monopolar energy generators, bipolar energy generators, dual electrosurgical/ultrasonic energy generators, display screens, and various other modules and/or other components described elsewhere herein.

Referring now to FIG. 4A, the header module 202 can, in some aspects, include the display screen 206 that renders a GUI 408 for relaying information regarding the modules 201 (FIG. 2) connected to the header module 202. In some aspects, the GUI 408 of the display screen 206 can provide a consolidated point of control of all of the modules 201 making up the particular configuration of the modular surgical system 200. In alternative aspects, the header module 202 can lack the display screen 206, or the display screen 206 can be detachably connected (removably attachable) to a housing 410 of the header module 202. In such aspects, the header module 202 can be communicably couplable to an external system that is configured to display the information generated by the modules 201 of the modular surgical system 200. For example, in robotic surgical applications, the modular surgical system 200 can be communicably couplable to a robotic cart or robotic control console, which is configured to display the information generated by the modular surgical system 200 to the operator of the robotic surgical system. As another example, the modular surgical system 200 can be communicably couplable to a mobile display that can be carried or secured to a surgical staff member for viewing thereby. In aspects utilizing a user interface that is separate from or otherwise distinct from the modular surgical system 200, the user interface can be wirelessly connectable with the modular surgical system 200 as a whole or one or more modules 201 thereof such that the user interface can display information from the connected modules 200 thereon.

Referring still to FIG. 4A, the energy module 204 can include a port assembly 412 including (providing) a number of different ports configured to deliver different energy modalities to corresponding surgical instruments (e.g., surgical instruments 300, 330, 360 of FIG. 3, for example) that are connectable thereto. In the particular aspect illustrated in FIGS. 4A, the port assembly 412 includes a bipolar port 414, a first monopolar port 416a, a second monopolar port 416b, a neutral electrode port 418 (to which a monopolar return pad is connectable), and a combination energy port 420. However, this particular combination of ports is simply provided for illustrative purposes and alternative combinations of ports and/or energy modalities may be possible for the port assembly 412.

As noted above, the modular surgical system 200 can be assembled into different configurations. Further, the different configurations of the modular surgical system 200 can also be utilizable for different surgical procedure types and/or different tasks. For example, FIGS. 4A and 4B illustrate a first illustrative configuration of the modular surgical system 200 including the header module 202 (including the display screen 206) and the energy module 204 connected together. Such a configuration can be suitable for laparoscopic and open surgical procedures, for example. As shown in FIG. 4B, the modular surgical system 200 can be positioned on a cart 230 enabling the modular surgical system 200 to be easily moved (wheeled) around the operating room, for example.

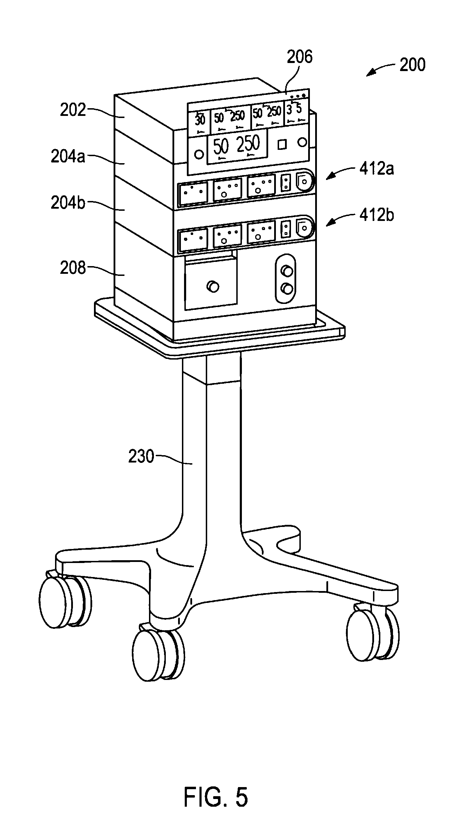

FIG. 5 illustrates a second illustrative configuration of the modular surgical system 200 including the header module 202 (including the display screen 206), a first energy module 204a, a second energy module 204b, and the evacuation module 208 connected together and positioned on the cart 230. In such a configuration, the evacuation module 208 can evacuate smoke, fluid, and/or particulates generated by surgical instruments powered by the energy modules 204a,b.

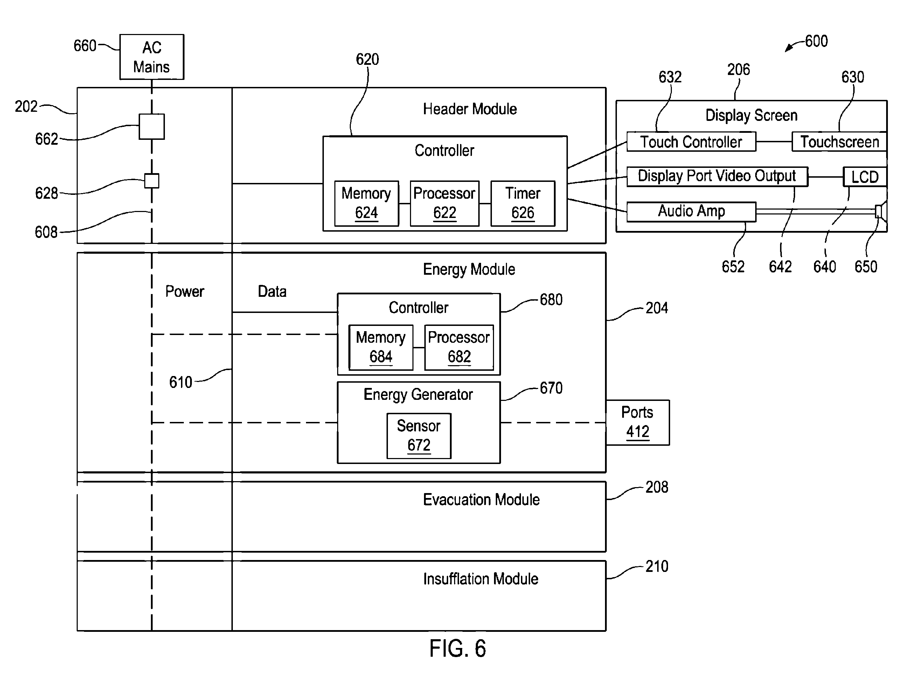

FIG. 6 is a block diagram of an example modular surgical system 600, in accordance with at least one aspect of the present disclosure. As illustrated, the modular surgical system 600 includes the header module 202 (including the display screen 206), the energy module 204 stacked under and coupled to the header module 202, the evacuation module 208 stacked under and coupled to the energy module 204, and the insufflation module 210 stacked under and coupled to the evacuation module 208.

The header module 202 is configured to monitor, control, energize, and provide feedback concerning operation of the modules within the modular surgical system 600, such as the energy module 204, the evacuation module 208, and the insufflation module 210. As illustrated, the header module 202 includes a controller 620 that comprises a processor 622 and a memory 624 storing computer readable instructions executable by the processor 622 to carry out functions and operations of the header module 602. Examples of the memory 624 include, but are not limited to, random access memory (RAM), read-only memory (ROM), computer chips, optical discs (e.g., compact discs (CDs), digital video discs (DVDs), etc.), magnetic disks (e.g., hard disk drives (HDDs), floppy disks, ZIP^{®} disks, etc.), magnetic tape, and solid state storage devices (e.g., memory cards, "flash" media, etc.). As used herein, the term "computer readable medium" refers to any device or system for storing and providing information (e.g., data and instructions) to the processor 622. Examples of computer readable media include, but are not limited to, optical discs, magnetic disks, magnetic tape, solid-state media, and servers for streaming media over networks.

Based on instructions stored in the memory 624, the processor 622 may be configured to control power and data transmissions between the header module 202, the energy module 204, the evacuation module 208, and the insufflation module 210 through a power interface 608 and a data interface 610. For example, the header module 202 can transmit various commands to the energy module 204, the evacuation module 208 (through the energy module 204), and the insufflation module 210 (through the energy module 204 and the evacuation module 208) via the data interface 610. Such commands can be based on user inputs received at the display screen 206 or inputs received by the controller 620 from various sensors communicably coupled to the modular surgical system 600, as discussed elsewhere herein.

As a further example, power may be transmitted to the energy module 204, the evacuation module 208 (through the energy module 204), and the insufflation module 210 (through the energy module 204 and the evacuation module 208) from the header module 202 via the power interface 608. The header module 202 may receive power from an external power source 660 (referred to herein as "AC Mains"), such as a wall outlet, for example. The header module 202 may include an AC/DC converter 662 which receives the AC power from the AC Mains 660 and converts the AC power to DC power. The controller 202 may then distribute the DC power to the energy module 204, the evacuation module 208, and the insufflation module 210 via the power interface 608. The controller 620 may further include a timer 626 for measuring elapsed time. The header module 202 may include a sensor 628, such as a current sensor and/or a power sensor, for example, in operable communication with the controller 620 for measuring current and power along the power interface 608.

As shown in FIG. 6, the energy module 204 may include a controller 680 that comprises a processor 682 and a memory 684 storing computer readable instructions executable by the processor 682 to carry out functions and operations of the energy module 204. The processor 682 and a memory 684 may be similar to processor 622 and memory 624, respectively. The controller 680 may receive power from the AC/DC converter 662 along the power interface 608 and may be in operable communication with controller 620 via the data interface 610.

The energy module 204 may further include an energy generator 670. The energy generator 670 may receive power from the AC/DC converter 662 along the power interface 608 and may be in operable communication with controller 680, such as via a wired or wireless connection. The energy generator 670 may be operable to provide therapeutic energy to one or more surgical instruments, such as the surgical instruments 300, 330, 360, via the port assembly 412, such as via the bipolar port 414 (FIG. 4), the first or second monopolar ports 416a, 416b (FIG. 4), or the combination energy port 420 (FIG. 4), for example. For instance, the energy generator 670 may be energized with DC power provided thereto from the AC/DC converter 662 along the power interface 608. The controller 680 may then receive an input, such as from the controller 620. Based on the input, the controller 680 may control the energy generator 670 to provide therapeutic energy to one or more surgical instruments coupled to the energy module 204 at the port assembly 412. The energy generator 670 may include a sensor 672, such as a current sensor and/or a power sensor, for example, in operable communication with the controller 680 for measuring current and/or power provided by the energy generator 670. The sensor 672 may also comprise an impedance sensor for measuring the impedance of tissue grasped by one of the surgical instruments.

As shown in FIG. 6, the display screen 206 includes a touchscreen 630 coupled to a touch controller 632. The touch controller 630 is coupled to the controller 620 to read inputs, such as user inputs, from the touchscreen 630. The controller 620 drives an LCD display 640 through a display/port video output signal 642. The controller 620 is further coupled to an audio amplifier 652 to drive one or more speakers 650.

### Surgical Insufflation

Minimally invasive surgical procedures often require the creation of a gas-filled cavity to provide surgeons with adequate visibility and space to manipulate instruments, such as the energy delivery devices 300, 330, 360 of FIG. 3. This gas-filled cavity may be generated using an insufflation module, such as insufflation module 210 (FIG. 2).

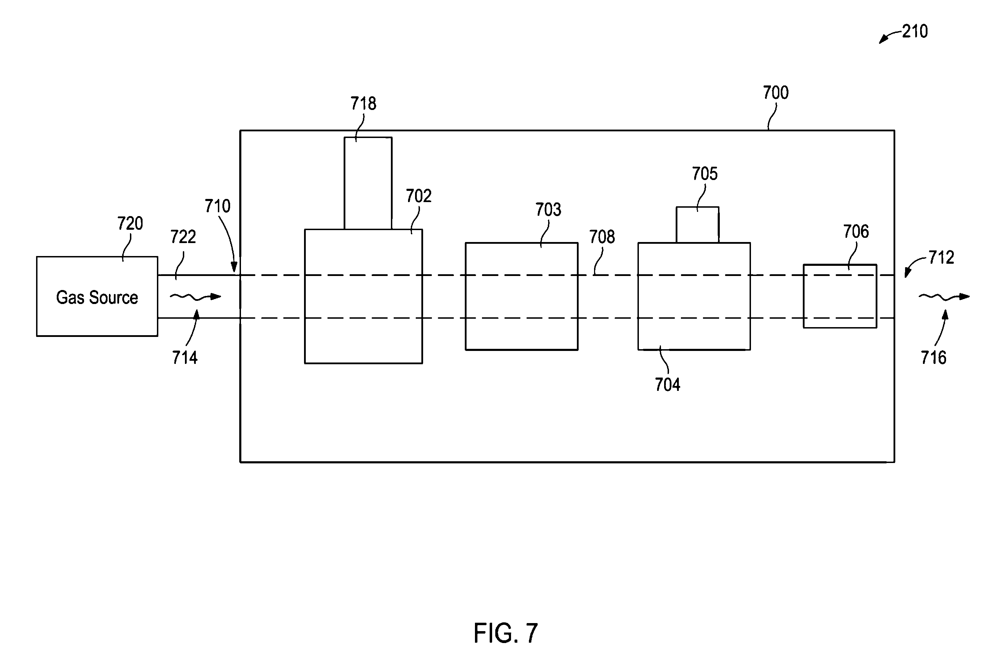

FIG. 7 is a schematic view of the internal components of the insufflation module 210 of FIG. 2, according to at least one aspect of the present disclosure. The insufflation module 210 may include an insufflation housing 700 that contains a fan or pump 702, a humidifier 703, a heat reservoir 704, a heater 705, and an exhaust mechanism 706, all positioned therewithin. In some embodiments, the heater 705 may be operable to generate heat and the heat reservoir 704 is configured to receive and store the heat generated by the heater 705. This stored heat may be used to passively warm gas that moves through the insufflation module 210, as will be described in more detail below. Alternatively, in other embodiments, the heat reservoir 704 may be omitted and the heater 705 may be operable to actively warm gas that moves through the insufflation module 210. The humidifier 703 may be operable to humidify the gas that moves through the insufflation module 210, as will also be described in more detail below. A motor 718 is provided to drive the pump 702. When the insufflation module 210 is stacked with the header module 202, similar to the arrangement shown in FIG. 6, the motor 718, the humidifier 703, and the heater 705 may receive power from the AC Mains 660 via the power interface 608.

The insufflation module 210 defines a flow path 708 (shown partially in dashed lines) extending through the insufflation housing 700 and having an inlet port 710 and an outlet port 712. The pump 702, the humidifier 703, the heat reservoir 704, and the exhaust mechanism 706 are sequentially arranged in-line within the flow path 708 through the insufflation housing 700 between the inlet port 710 and the outlet port 712. In some embodiments, the heat reservoir 704 is a ring that encircles the flow path 708 to passively warm gas that moves through the flow path 708. The outlet port 712 may be fluidically coupled to a trocar, which may be in fluid communication with an internal cavity of a patient (e.g., a patient's peritoneal cavity).

The inlet port 710 may be fluidically coupled to a gas source 720 with a conduit 722 (tubing). The gas source 720 may contain a gas, such as carbon dioxide (CO₂), nitrous oxide (N₂O), helium, oxygen, air, xenon, argon, or nitrogen (N₂), as examples.

The pump 702 is configured to produce a pressure differential in the flow path 708 by mechanical action. The pressure differential draws gas 714 from the gas source 720 through the conduit 722, into the inlet port 710, and along the flow path 708. After moving through the humidifier 703 and the heat reservoir 704, the gas 714 can be considered "heated/humidified" gas 716 (referred to herein as "insufflation" gas 716), which can continue through the flow path 708 and the exhaust mechanism 706, eventually being expelled (discharged) through the outlet port 712. The exhaust mechanism 706 may control the velocity, the direction, and/or other properties of the insufflation gas 716 exiting the insufflation module 210 at the outlet port 712.

The flow path 708 through the insufflation module 210 can be comprised of a tube or other conduit that substantially contains and/or isolates the fluid moving through the flow path 708 from the fluid (the ambient environment) outside the flow path 708.

### Surgical Smoke Evacuation

As provided herein, energy devices, such as the energy delivery devices 300, 330, 360 of FIG. 3, deliver mechanical (ultrasonic, for example) and/or electrical (RF, for example) energy to target tissue in order to treat the tissue (e.g. to cut the tissue, cauterize blood vessels and/or coagulate the tissue within and/or near the targeted tissue). Cutting, cauterization, and/or coagulation of tissue can result in fluids and/or particulates being released into the air. Such fluids and/or particulates emitted during a surgical procedure can constitute smoke, for example, which can comprise carbon particles and/or other particles suspended in air. In other words, a fluid can comprise smoke and/or other fluidic matter.

Approximately 90% of endoscopic and open surgical procedures generate some level of smoke. The smoke can be unpleasant to the olfactory senses of the clinician(s), the assistant(s), and/or the patient(s), may obstruct the clinician(s)'s view of the surgical site, and may be unhealthy to inhale in certain instances. For example, smoke generated during an electrosurgical procedure can contain toxic chemicals including acrolein, acetonitrile, acrylonitrile, acetylene, alkyl benzenes, benzene, butadiene, butene, carbon monoxide, creosols, ethane, ethylene, formaldehyde, free radicals, hydrogen cyanide, isobutene, methane, phenol, polycyclic aromatic hydrocarbons, propene, propylene, pyridene, pyrrole, styrene, toluene, and xylene, as well as dead and live cellular material (including blood fragments), and viruses. Certain materials identified in surgical smoke have been classified as containing known carcinogens. It is estimated that one gram of tissue cauterized during an electrosurgical procedure can be equivalent to the toxins and carcinogens of six unfiltered cigarettes. Additionally, exposure to the smoke released during an electrosurgical procedure has been reported to cause eye and lung irritation to health care workers.

In addition to the toxicity and odors associated with the material in surgical smoke, the size of particulate in surgical smoke can be harmful to the respiratory system of the clinician(s), the assistant(s), and/or the patient(s). In certain instances, the particulates can be extremely small, and repeated inhalation of extremely small particulate can lead to acute and chronic respiratory conditions in certain instances.

Many electrosurgical systems employ a surgical evacuation system that draws in and captures the resultant smoke from a surgical procedure, and directs the captured smoke through a filter and an exhaust port away from the clinician(s) and/or from the patient(s). For example, an evacuation system, such as the evacuation module 208 (FIG. 2), can be configured to evacuate smoke that is generated during an electrosurgical procedure. Such an evacuation system can be referred to as a "smoke evacuation system," though such evacuation systems can be configured to evacuate more than just smoke from a surgical site.

Throughout the present disclosure, the "smoke" evacuated by an evacuation system is not limited to just smoke. Rather, the smoke evacuation systems disclosed herein can be used to evacuate a variety of fluids, including liquids, gases, vapors, smoke, steam, or combinations thereof. The fluids can be biologic in origin and/or can be introduced to the surgical site from an external source during a procedure. The fluids can include water, saline, lymph, blood, exudate, and/or pyogenic discharge, for example. Moreover, the fluids can include particulates or other matter (e.g. cellular matter or debris) that is evacuated by the evacuation system. For example, such particulates can be suspended in the fluid.

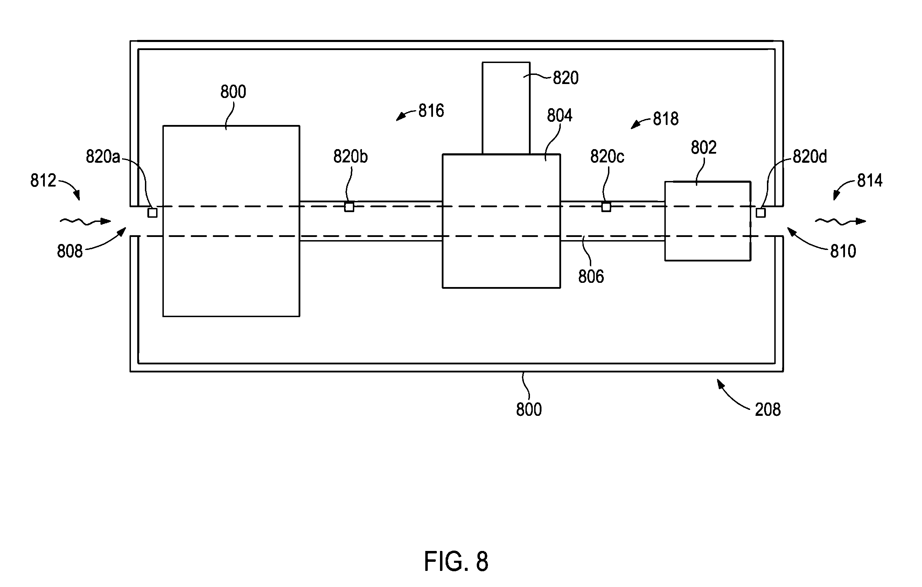

FIG. 8 is a schematic view of the internal components of the evacuation module 208 of FIG. 2, according to at least one aspect of the present disclosure. The evacuation module 208 includes an evacuation housing 801 with a fan or pump 804 and a filter 800 positioned therewithin. Smoke drawn into the evacuation housing 801 travels to the filter 800, and harmful toxins and offensive smells are filtered out of the smoke as it moves through (traverses) the filter 800. Filtered air 814 may then exit the evacuation module 208 as exhaust.

The evacuation module 208 defines a flow path 806 (shown in dashed lines) extending through the evacuation housing 801 and having an inlet port 808 and an outlet port 810. The filter 800, the pump 804, and the exhaust mechanism 802 are sequentially arranged in-line within the flow path 806 through the evacuation housing 801 between the inlet port 808 and the outlet port 810. The inlet port 808 may be fluidically coupled to a suction conduit 702, which can comprise a distal conduit opening positionable at a surgical site.

The pump 804 is configured to produce a pressure differential in the flow path 806 by a mechanical action. The pressure differential is configured to draw smoke 812 from the surgical site into the inlet port 808 and along the flow path 806. After moving through the filter 800, the smoke 812 can be considered "filtered" smoke or air 814 (referred to herein as "filtered air 814"), which can continue through the flow path 806 and is eventually expelled (discharged) through the outlet port 810.

As illustrated, the flow path 806 may include a first zone 816 and a second zone 818. The first zone 816 is located upstream from the pump 804; the second zone 818 is located downstream from the pump 804. The pump 804 is configured to generate the vacuum and otherwise pressurize the fluid in the flow path 806 to drive the fluid from the first zone 816 to the second zone 818 through the pump 804. A motor 820 drives the pump 804. When the evacuation module 208 is stacked with the header module 202, similar to the arrangement shown in FIG. 6, the motor 820 may receive power via the power interface 608. The exhaust mechanism 802 is a mechanism that can control the velocity, the direction, and/or other properties of the filtered air 814 exiting the evacuation module 208 at the outlet port 810.

The flow path 806 through the evacuation module 208 can be comprised of a tube or other conduit that substantially contains and/or isolates the fluid moving through the flow path 806 from the fluid (the ambient environment) outside the flow path 806. For example, the first zone 816 of the flow path 806 can comprise a tube through which the flow path 806 extends between the filter 800 and the pump 804. The second zone 818 of the flow path 806 can also comprise a tube (conduit) through which the flow path 806 extends between the pump 804 and the exhaust mechanism 802. The flow path 806 also extends through the filter 800, the pump 804, and the exhaust mechanism 802 so that the flow path 806 extends continuously from the inlet port 808 to the outlet port 810.

In operation, the smoke 812 can flow into the filter 800 after traversing the inlet port 808 and can be pumped through the flow path 806 by the pump 804 such that the smoke 812 is drawn into the filter 800. The filtered air 814 discharged from the filter 800 can then be pumped through the exhaust mechanism 802 and out the outlet port 810 of the evacuation module 208. The filtered air 814 exiting the evacuation module 208 at the outlet port 810 is the exhaust, and can consist of filtered gases that have passed through the evacuation module 208. Additional information regarding the evacuation module 208 is described in U.S. Patent No. 11,602,393, entitled "SURGICAL EVACUATION SENSING AND GENERATOR CONTROL", issued March 14, 2023, which is hereby incorporated by reference in its entirety herein.

According to embodiments of the present disclosure, the evacuation module 208 may further include a plurality of sensors 820a, 820b, 820c, 820d positioned along the flow path 806 for measuring one or more parameters associated with the smoke 812 and/or the filtered air 814 flowing along the flow path 806. When the evacuation module 208 is in a stacked configuration with a header module 202, such as in the configuration shown in FIG. 6, for example, the sensors 820a-d can be in operable communication with the controller 620, such as along the data interface 610 (FIG. 6), and can receive power from the header module 202 via the power interface 608 (FIG. 6). The controller 620 may receive measurements from the sensors 820a-d and control various operations of the modular surgical system based on these measurements. For example, in some embodiments, the controller 620 can control a speed of the motor 820 based on the received measurements.

One or more of the sensors 820a-d may comprise flow sensors for measuring a flow rate of the smoke 812/filtered air 814 along the flow path 806, such as a flow rate entering the inlet port 808 (sensor 820a), a flow rate through the first zone 816 (sensor 820b), a flow rate through the second zone 818 (sensor 820c), and/or a flow rate exiting the outlet port 810 (sensor 820d), for example. Alternatively, or in addition thereto, one or more of the sensors 820a-d may comprise pressure sensors for measuring a pressure of smoke 812/filtered air 814 along the flow path 806, such as at the inlet port 808 (sensor 820a), the first zone 816 (sensor 820b), the second zone 818 (sensor 820c), and/or at the exit outlet port 810 (sensor 820d), for example. In some embodiments, the sensors 820a-d may be used by the controller 620 to measure a pressure differential along the flow path 806, such as a pressure differential across the filter 800 using the first and second sensors 820a,b. The evacuation module 208 may include a combination of both flow sensors and pressure sensors.

### Intelligent Power Control Insufflation functionality based on smoke evacuator operations

Due to the varying number of modules utilized, a modular surgical system may require power beyond what is available from a single AC Mains 660, such as two or three AC Mains, each requiring a dedicated branch circuit in the operating room. By intelligently utilizing and distributing power from the single AC Mains 660, a reduction of the total necessary peak demand on the AC Mains 660 may occur, which may help reduce constraints on the types of combined modules (*e.g.*, header module, energy modules, evacuation modules, insufflation modules, *etc.*) and for the total number of AC Mains connections for the modular surgical system.

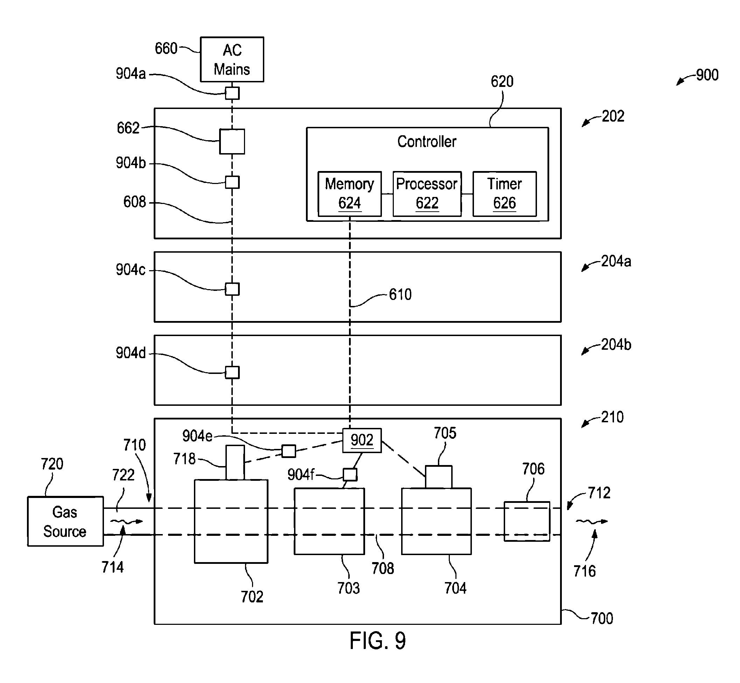

FIG. 9 is a block diagram of a modular surgical system 900, in accordance with at least one aspect of the present disclosure. As illustrated, the modular surgical system 900 includes a header module 202 (which may include a display screen 206 (FIG. 6)), a first energy module 204a stacked under and coupled to the header module 202, a second energy module 204b stacked under and coupled to the first energy module 204a, and an insufflation module 210 stacked under and coupled to the second energy module 204b. The modular surgical system 900 may include additional modules, described elsewhere herein, such as an evacuation module 208 (FIG. 6), for example, or may include less modules (e.g., only one energy module 204). The modular surgical system 900 may be similar in some respects to the modular surgical system 600.

The energy modules 204a,b are significant users of the AC Mains 660 power in the module energy system 900. Each of the energy modules 204a,b may be transitionable between an active state, in which the energy module 204a,b transmits energy to a surgical instrument (e.g., one of surgical instruments 300, 330, 360 of FIG. 3), and an idle state, in which the energy module 204a,b abstains from transmitting energy to a surgical instrument. As will be appreciated, the energy modules 204a,b consume less power in the idle state as opposed to in the active state. For instance, in the idle state, the header module 202 and the two energy modules 204a,b may consume a first amount of power (e.g., 275W; 137.5W per energy module), and while in the active states, the header module 202 and the two energy modules 204a,b may be constrained to consume up to a second amount of power (e.g., 785W; 392.5W per energy module) which is greater than the first amount of power.

In real world scenarios, the actual time when the energy modules 204a,b are in the active state is significantly less than when the energy modules 204a,b are in the idle state. Furthermore, in most situations, the energy modules 204a,b do not deliver maximum energy, rather they may be constrained to deliver only a fraction (percentage) of the maximum energy.

The AC Mains 660 may be capable of delivering a source amount of power (e.g. 1050W) to the modular surgical system 900. Accordingly, with both energy modules 204a,b in their active states, a first free amount of power may be available for the remainder of the module energy system 900 *(e.g.,* 1050W - 785W = 265W), and with the energy modules 204a,b in their idles states, a second free amount of power may be available for the remaining modules of the module energy system 900 *(e.g.,* 1050W - 275W = 775W) which is greater than the first amount of free power. The amount of free power may also vary based on other modules in the modular surgical system 900 being transitioned between idle and active states as well. For example, the modular surgical system 900 may further include an evacuation module 208 (FIG. 8) and the amount of free power may further be based on whether the evacuation module 208 is in an active state, in which the pump 804 (FIG. 8) draws smoke 812 (FIG. 8) into the evacuation module 208, and an idle state, in which the pump 804 abstains from drawing smoke 812 into the evacuation module 208.

As discussed elsewhere herein, the insufflation module 210 includes a pump 702 that is configured to draw gas 714 from the gas source 720 through the conduit 722, into the inlet port 710, along the flow path 708, and through the humidifier 703 and the heat reservoir 704, thereby generating insufflation gas 716. Operating the insufflation module 210 may require a first amount of power (*e.g.*, 200W) without heating the gas 714 with the heat reservoir 704 / heater 705. For instance, the insufflation module 210 may require the first amount of power to activate the motor 718 to draw the gas 714 therein and/or energize the humidifier 703. Furthermore, the insufflation module 210 may be constrained to consume up to a second amount of power (*e.g.*, 850W) greater than the first amount of power when heating the gas 714 with the heat reservoir 704 / heater 705.

As discussed above, the amount of power available for remaining modules of the modular surgical system 900 is based on whether the energy modules 204a,b are in their active or idle states. For instance, with both energy modules 204a,b in their active state, there is a first free amount of power available for the insufflation module 210 (*e.g.*, 265W). Accordingly, with the energy modules 204a,b in their active state, a first amount of thermal energy is available for heating the gas 714 (*e.g.*, 265W - 200W = 65W). Furthermore, with the energy modules 204a,b in their idle state, there is a second free amount of power available for the insufflation module 210 (*e.g.,* 775W). Accordingly, with the energy modules 204a,b in their idle state, a second amount of thermal energy greater than the first amount of thermal energy is available for heating the gas (*e.g.*, 775W - 200W = 575W).

It should be noted that the power values provided above are merely illustrative, demonstrating boundary conditions for the modular surgical system 900, and illustrate how delivery of less energy to a patient via the energy modules 204a,b results in more available energy for the insufflation module 210 to heat the gas from the gas source 720.

As discussed above, by intelligently utilizing and distributing power from the single AC Mains 660, a reduction of the total necessary peak demand on the AC Mains 660 may occur. Accordingly, the insufflation module 210 may further include a power controller 902 that may be in operable communication with the AC Mains 660 via the power interface 608 and may in operable communication with the other modules in the modular surgical system 900 via the data interface 610.

The power controller 902 may be operable to determine a free amount of power available from the AC mains 660. The free amount of power may be the difference between the source amount of power from the AC Mains 660 and the amount of power being consumed by the other modules in the modular surgical system 900.

The modular surgical system 900 may further include a power sensor 904a for sensing the source amount of power from the AC Mains 660 and a plurality of power sensors 904b, 904c, 904d, 904e, 904f for sensing the amount of power being drawn from the various other modules in the modular surgical system 900. For instance, power sensor 904b may sense a power draw of the header module 202 from the AC Mains 660, power sensors 904c,d may sense a power draw of the energy modules 202 from the AC Mains 660, power sensor 904e may sense a power draw of the motor 718 from the AC Mains 660, and power sensor 904f may sense a power draw of the humidifier 703. The power controller 902 may communicate with the power sensors 900a-f via one or more wired connections, such as along the data interface 610, or wirelessly. The power sensors 904a-f may be current sensors, voltage sensors, or any other suitable sensor that is designed to sense power draw.

The power controller 902 may be operable to determine an amount of free power available from the AC Mains 660 by subtracting the sensed power draws, as sensed by power sensors 904b-f, from the sensed source amount of power from the AC Mains 660, as sensed by power sensor 904a. Based on determining the amount of free power available from the AC Mains 660, the power controller 902 may draw up to the free amount of power from the AC Mains 660 to power the heater 705, thereby storing heat in the heat reservoir 704.

For example, as discussed above, the AC Mains 660 may be capable of delivering 1050W of power to the modular surgical system 900. Both energy modules 204a,b may be transitioned to their active states, which may utilize 785W of power from the AC Mains 660. The power controller 902 may detect the source amount of power of the AC Mains 660 via the power sensor 904a and the amount of power drawn by the header module 202 and energy modules 204a,b via the power sensors 904b-d. Based on the sensed readings from the sensors 904b-d, the power controller 902 may determine that 265W of free power is available for the insufflation module 210. Accordingly, the power controller 902 may draw up to the free 265W of power and distribute 200W to the motor 718 and the humidifier 703 and the remaining 65W to the heater 705 to heat the heat reservoir 704.

At a later time, both energy modules 204a,b may be transitioned to their idles states, which may now utilize 275W of power from the AC Mains 660. The power controller 902 may detect this change via the power sensors 904b-d and, based on the sensed readings from the sensors 904b-d, the power controller 902 may determine that 775W of free power is available from the AC Mains 660 for the insufflation module 210. Accordingly, the power controller 902 may draw up to the free 775W of power and distribute 200W to the motor 718 and the humidifier 703 and the remaining 575W to the heater 705 to heat the heat reservoir 704.

Accordingly, the foregoing arrangement enables the modular surgical system 900 to dynamically and intelligently change the amount of thermal energy that is provided to the heater 705 based on an amount of available power from the AC Mains 660, thereby reducing the total necessary peak demand from the AC Mains 660 and potentially reducing the constraints on the types of combined modules and for the total number of AC Mains connections for the modular surgical system 900.

While the foregoing description was with respect to a power controller 902 that may draw a free amount of power from an AC Mains 660 for an insufflation module 210, other embodiments are envisioned in which other modules include the power controller 902 for drawing free power. For instance, in some embodiments, the evacuation module 208 (FIG. 8) may include the power controller 902 for drawing free power for powering the motor 820 (FIG. 8). In such embodiments, the evacuation module 208 may dynamically adjust the speed of the pump 804 (FIG. 8) according to the amount of free power from the AC Mains 660.

In some embodiments, the power controller 902 may determine an amount of free power from the AC Mains 660 and intelligently distribute the power between a plurality of modules. For instance, the power controller 902 may determine that a free amount of power is available from the AC Mains 660 and selectively distribute a first amount of the free power to a first surgical module, such as the insufflation module 210, and a second amount of the free power to a second surgical module, such as an evacuation module 208. Accordingly, the power controller 902 can intelligently utilize the free amount of power from the AC Mains 660 for a plurality of modules.

Alternatively, the modular surgical system 900 may not include a power controller 902 and, rather, the controller 620 of the header module 202 may perform all the aforementioned functions of the power controller 902. For instance, the sensors 904a-f may be in operable communication with the controller 620 and the controller 620 can selectively distribute the free amount of power to the insufflation module 210, as discussed above.

### Pressure Chamber Air Management

As discussed above, a modular surgical system may require power beyond what is available from a single AC Mains, such as two or three AC Mains, each requiring a dedicated branch circuit in the operating room due to the varying number of modules that may be utilized. By intelligently utilizing and distributing power from the single AC Mains, a reduction of the total necessary peak demand on the AC Mains may occur, which may help reduce constraints on the types of combined modules and for the total number of AC Mains connections for the modular surgical system.

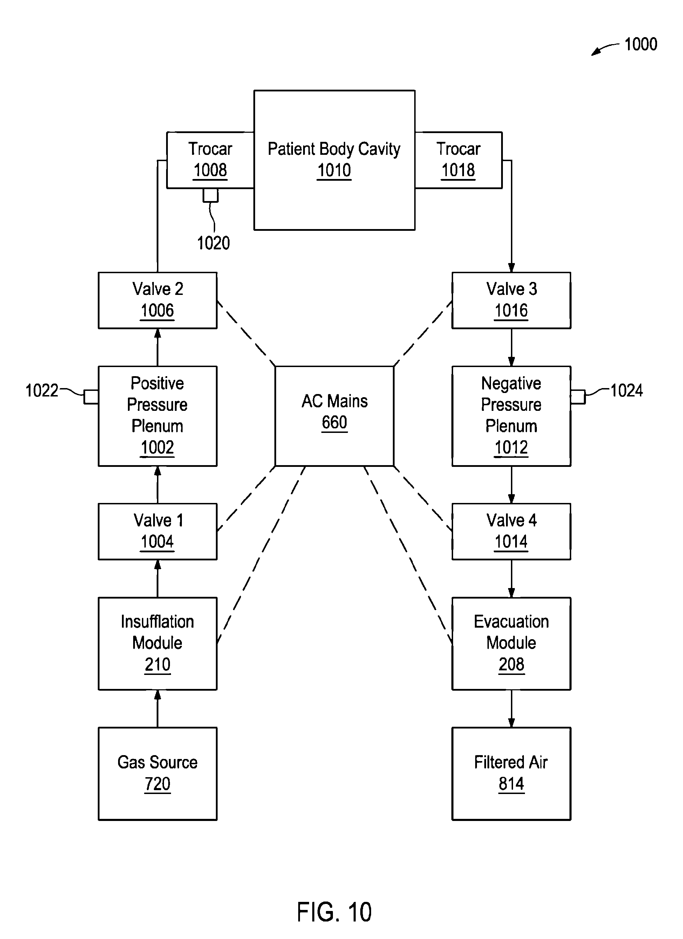

FIG. 10 is a block diagram of a modular surgical system 1000, in accordance with at least one aspect of the present disclosure. The modular surgical system 1000 may be similar in arrangement and function to the modular surgical system 600 of FIG. 6 and therefore may be best understood with reference thereto, where like numerals will be used to represent like components that are not described again in detail.

The modular surgical system 1000 may include an insufflation module 210, a gas source 720 fluidically coupled to the insufflation module 210, such as via a conduit 722 (FIG. 7), and a positive pressure plenum 1002 fluidically coupled to the insufflation module 210. The modular surgical system 1000 may further include a first valve 1004 positioned between the insufflation module 210 and the positive pressure plenum 1002 and that is transitionable between an open state, where the insufflation module 210 and the positive pressure plenum 1002 fluidly communicate, and a closed state, where fluid communication between the insufflation module 210 and the positive pressure plenum 1002 is prevented.

The modular surgical system 1000 may further include a second valve 1006 positioned between the positive pressure plenum 1002 and an insufflation trocar 1008 inserted into a patient's body cavity 1010 (*e.g.,* a patient's peritoneal cavity) and that is transitionable between an open state, to fluidically couple the positive pressure plenum 1002 and the patient's body cavity 1010, and a closed state, to fluidically decouple the positive pressure plenum 1002 from the patient's body cavity 1010.

The modular surgical system 1000 may further include an evacuation module 208 and a negative pressure plenum 1012 fluidically coupled to the evacuation module 208, such as via tubing, like conduit 722 (FIG. 7). The modular surgical system 1000 may further include a third valve 1016 positioned between the negative pressure plenum 1012 and an evacuation trocar 1018 inserted into the patient's body cavity 1010 (*e.g.,* a patient's peritoneal cavity) and that is transitionable between an open state, to fluidically couple the negative pressure plenum 1012 and the patient's body cavity 1010, and a closed state, to fluidically decouple the negative pressure plenum 1012 from the patient's body cavity 1010.

The modular surgical system 1000 may further include a fourth valve 1014 positioned between the evacuation module 208 and the negative pressure plenum 1012 and that is transitionable between an open state, to fluidically couple the evacuation module 208 and the negative pressure plenum 1012, and a closed state, to fluidically decouple the evacuation module 208 from the negative pressure plenum 1012.

The valves 1004, 1006, 1014, 1016 may be any suitable valve (e.g., gate, globe, ball, etc.) that is transitionable (actuatable) between open and closed states. The valves 1004, 1006, 1014, 1016 may each include a motor in operable communication with the controller 620 (FIG. 6) and the AC Mains 660, which function to transition the valves 1004, 1006, 1014, 1016 between their respective open and closed states. Alternatively, the valves 1004, 1006, 1014, 1016 may be solenoid valves that are transitionable between their respective open and closed states by the controller 620 and powered by the AC Mains 660. The trocars 1008, 1018 may be similar to the trocars described elsewhere herein, or the trocars described in U.S. Patent No. 11,369,443, titled "METHOD US USING A SURGICAL MODULAR ROBOTIC ASSEMBLY", which issued on June 28, 2022, the contents of which are hereby incorporated by reference in their entirety herein.

Referring to FIGS. 6 and 10, the controller 620 may function to determine a free amount of power available from the AC mains 660, as described elsewhere herein. The free amount of power may be the difference between the source amount of power from the AC Mains 660 and the power being consumed by the other modules in the modular surgical system 1000.

For instance, the modular surgical system 1000 may further include a power sensor (similar to power sensor 904a of FIG. 9) for sensing the source amount of power from the AC Mains 660 and a plurality of power sensors (similar to power sensors 904b-f of FIG. 9) for sensing the amount of power being drawn from the various other modules in the modular surgical system 900. The controller 620 may communicate with the power sensors via one or more wired connections, such as along the data interface 610, or wirelessly. The power sensors may be current sensors, voltage sensors, or any other suitable sensor that is designed to sense power draw.

The modular surgical system 1000 may further include a pressure sensor 1020 for sensing a pressure of the patient's body cavity 1010. The pressure sensor 1020 may be fluidically coupled to the patient's body cavity 1010 at the insufflation trocar 1008, at the evacuation trocar 1018, or at any other suitable location. The modular surgical system 1000 may further include pressure sensors 1022, 1024 for sensing the pressure within the positive and negative pressure plenums 1002, 1012, respectively.

The controller 620 may be operable to determine an amount of free power available from the AC Mains 660 by subtracting the sensed power draws from the sensed source amount of power from the AC Mains 660. The controller 620 may compare the determined amount of free power to a power threshold, which may be stored in the memory 624. Based on the comparison, the controller 620 may selectively control the valves 1004, 1006, 1014, 1016, the insufflation module 210, and the evacuation module 208, as will be described in more detail below.

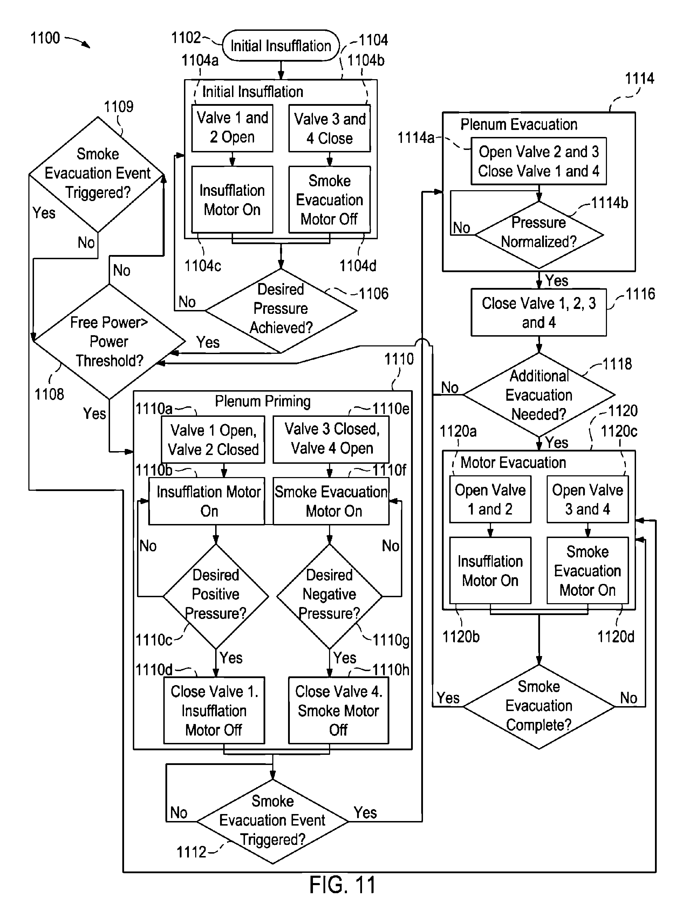

Referring now to FIG. 11, illustrated is a flow diagram 1100 for controlling the modular surgical system 1000 of FIG. 10, according to at least one aspect of the present disclosure. In some embodiments, the flow diagram 1100 is embodied as an algorithm, stored in the memory 624 (FIG. 6) of the controller 620 (FIG. 6), and is executable by the processor 622 (FIG. 6).

Discussion of the flow diagram 1100 is now provided, with reference to FIGS. 6-8 and 10. At step 1102, the controller 620 may execute the algorithm 1100, such as in response to a user input at the touchscreen 630, or the modular surgical system 1000 being powered on by the AC Mains 660.

The controller 620 may then proceed to step 1104, at which the controller 620 provides initial insufflation to the patient's body cavity 1010. More specifically, the controller 620 transitions (places) the valves 1004, 1006 to their respective open states (step 1104a), transitions the valves 1014, 1014 to their respective closed states (step 1104b), energizes the insufflation motor 718 (step 1104c), and maintains the evacuation motor 820 in a de-energized, or idle, state (step 1104d). Accordingly, at step 1104, the controller 620 drives gas 714, with the insufflation motor 718, from the gas source 720 through the insufflation module 210 (thereby generating insufflation gas 716), through the valve 1004, the positive pressure plenum 1002, the valve 1006, the insufflation trocar 1008, and into the patient's body cavity 1010, thereby providing initial insufflation to the patient's body cavity 1010.

The controller 620 may then proceed to step 1106, at which the controller 620 determines if a desired pressure has been achieved in the patient's body cavity 1010. For instance, the controller 620 may interrogate the pressure sensor 1020, such as along the data interface 610 or wirelessly, to determine the pressure of the patient's body cavity 1010. The controller 620 may retrieve from the memory 624 a pressure threshold and compare the determined pressure of the patient's body cavity 1010 to the pressure threshold. If the pressure of the patient's body cavity 1010 is less than the pressure threshold, the controller 620 may proceed back to step 1104 to provide additional insufflation to the patient's body cavity 1010. In contrast, if the pressure of the patient's body cavity 1010 is at or greater than the pressure threshold, the controller 620 may proceed to step 1108.

At step 1108, the controller 620 may determine the amount of free power available from the AC Mains 660 and determine if the amount of free power is at or greater than a power threshold. For instance, as discussed above, the controller 620 may interrogate the various power sensors of the modular surgical system 1000 to determine the amount of free power from the AC Mains 660. The controller 620 may then compare the amount of free power to a power threshold, which may be stored in the memory 624, to determine if the amount of free power is at or greater than a power threshold.

Based on the controller 620 determining that the amount of free power is less than the power threshold, the controller 620 may loop and continue to determine when/if the amount of free power reaches/exceeds the power threshold. As the controller 620 loops and continues to determine when/if the amount of free power reaches/exceeds the power threshold, the controller 620 may determine if a smoke evacuation event has been triggered (detected) at step 1109. The smoke evacuation event may result from a user providing an input to touchscreen 630, the pressure patient's body cavity 1010 (as sensed by sensor 1020) reaching or exceeding a pressure threshold, which may be stored in the memory 624, or any other suitable event that would cause the motor 820 of the evacuation module 208 to energize to evacuate smoke 812 from the patient's body cavity 1010, as described elsewhere herein. If a smoke evacuation event is not triggered, the controller 620 may continue looping to determine when/if the amount of free power reaches/exceeds the power threshold. If a smoke evacuation event is triggered, the controller 620 may proceed to step 1120 to provide insufflation and evacuation to the patient's body cavity, as will be described in more detail below.

Based on the controller 620 determining that the amount of free power reaches or exceeds the power threshold, as at step 1108, the controller 620 may proceed to step 1110 to prime (pressurize) the positive and negative pressure plenums 1002, 1012. More specifically, the controller 620 may place the valve 1004 in the open state and place the valve 1006 in the closed state (step 1110a) and energize the insufflation motor 718 (step 1110b), thereby positively pressurizing the positive pressure plenum 1002. The controller 620 may then proceed to step 1110c at which the controller 620 determines if the desired pressure has been achieved at the positive pressure plenum 1002. For instance, the controller 620 may interrogate the pressure sensor 1022 to determine the pressure at the positive pressure plenum 1002 and compare the determined pressure to a positive pressure threshold, which may be stored in the memory 624. Based on the determined pressure being less than the positive pressure threshold, the controller 620 may proceed back to step 1110b and continue pressurizing the positive pressure plenum 1002. If the pressure reaches or exceeds the positive pressure threshold, the controller 620 may proceed to step 1110d, at which the controller 620 de-energizes the insufflation motor 718 and transitions the valve 1004 to the closed state, thereby generating a "pressurized" positive pressure plenum 1002.

Similarly, the controller 620 may place the valve 1014 in the open state and place the valve 1016 in the closed state (step 1110e) and energize the evacuation motor 820 (step 1110f), thereby negatively pressurizing the negative pressure plenum 1012. The controller 620 may then proceed to step 1110g at which the controller 620 determines if the desired pressure has been achieved at the negative pressure plenum 1012. For instance, the controller 620 may interrogate the pressure sensor 1024 to determine the pressure at the negative pressure plenum 1012 and compare the determined pressure to a negative pressure threshold, which may be stored in the memory 624. If the pressure is greater than the negative pressure threshold, the controller 620 may proceed back to step 1110f and continue pressurizing the negative pressure plenum 1012. If the pressure reaches or drops below the negative pressure threshold, however, the controller 620 may proceed to step 1110h, at which the controller 620 de-energizes the evacuation motor 820 and transitions the valve 1014 to the closed state, thereby generating a "pressurized" negative pressure plenum 1012.

During step 1110, the controller 620 may continuously, or periodically, compare the amount of free power from the AC Mains 660 to the power threshold as the plenums are primed. Based on the amount of free power dropping below the power threshold during step 1110, the controller 620 may advance to steps 1110d and 1110h, thereby halting priming of the positive and negative pressure plenums 1002, 1012. In some embodiments, the controller 620 may proceed back to step 1110 to continue priming the positive and negative pressure plenums 1002, 1012 based on the amount of free power again reaching or exceeding the power threshold and at least one of the positive and negative pressure plenums 1002, 1012 not reaching their respective positive and negative pressure thresholds.

In some embodiments, during step 1110, the controller 620 may pressurize the positive pressure plenum 1002 (steps 1110a-c) while also pressurizing the negative pressure plenum 1012 (steps 1110e-g). In other embodiments, during step 1110, the controller 620 may only pressurize one of the positive pressure plenum 1002 (steps 1110a-c) or the negative pressure plenum 1012 (steps 1110e-g). For example, during step 1110, the controller 620 may first utilize the available free power from the AC Mains 660 to pressurize the negative pressure plenum 1012 (steps 1110e-g). Once the negative pressure plenum 1012 has been sufficiently primed (step 1110g), the controller may proceed to step 1110h to de-energize the evacuation motor 820 and transitions the valve 1014 to the closed state, thereby generating a "pressurized" negative pressure plenum 1012. The controller 620 may then proceed to utilize the available free power from the AC Mains 660 to pressurize the positive pressure plenum 1002 (steps 1110a-c).

The controller 620 may then proceed to step 1112 and determine if a smoke evacuation event has been triggered (detected), similar to step 1109. If a smoke evacuation event has not being triggered, the controller 620 may loop at step 1112 until a smoke evacuation event is triggered, or may proceed back to step 1110 to provide additional priming to one or both of the positive and negative plenums 1002, 1012 if the respective positive and negative pressure thresholds have not been achieved and the controller 620 determines that the amount of free power from the AC Mains 660 is greater than the power threshold.

If the smoke evacuation event is triggered, the controller 620 may proceed to step 1114, at which the controller 620 may transition valves 1006, 1016 to their respective open states, maintaining valves 1004, 1014 in their closed states (step 1114a). Due to the generated positive pressure in the positive pressure plenum 1002 (at step 1110), the insufflation gas 716 within the positive pressure plenum 1002 is passively driven from the positive pressure plenum 1002, through the valve 1006, the insufflation trocar 1008, and into the patient's body cavity 1010. Similarly, due to the generated negative pressure in the negative pressure plenum 1012 (at step 1110), smoke 812 within the patient's body cavity 1010 is passively driven through the evacuation trocar 1018, through the valve 1016, and into the negative pressure plenum 1012.

The controller 620 may then proceed to step 1114b and determine if the pressure within the positive and negative pressure plenums 1002, 1012 have normalized. For instance, the controller 620 may interrogate the pressure sensors 1022, 1024 and determine if the sensed pressures at pressure sensors 1022, 1024 are identical, or at least substantially identical. The controller 620 may also interrogate the pressure sensor 1020 and determine if the sensed pressures at the pressure sensors 1020, 1022, 1024 are identical, or at least substantially identical.

Based on determining that the pressures within the positive and negative pressure plenums 1002, 1012 have not yet normalized, the controller 620 may loop at step 1114b until the pressures normalize. Once it is determined that the pressures within the positive and negative pressure plenums 1002, 1012 have normalized, the controller 620 may proceed to step 1116 and transition all the valves 1004, 1006, 1014, 1016 to their respective closed states. The controller 620 may then proceed to step 1118 and determine if additional evacuation is needed. The controller 620 may determine if evacuation is needed based on receiving a user input, such as at the touchscreen 630, determining that the pressure in the patient's body cavity 1010(as sensed by sensor 1020) is still at or above the pressure threshold, or any other suitable determination that would necessitate or require the motor 820 of the evacuation module 208 to be energized, as described elsewhere herein.

Based on the controller 620 determining that no additional smoke evacuation is needed, the controller 620 may proceed back to step 1108 and determine if the amount of free power of the AC Mains 660 is at or greater than the power threshold to attempt to reprime the positive and negative pressure plenums 1002, 1012. If the controller 620 determines that additional smoke evacuation is needed, the controller 620 may proceed to step 1120, at which the controller 620 places the valves 1004, 1006 in their respective open states (step 1120a) and energizes the insufflation motor 718 (step 1120b). Similarly, the controller 620 may place the valves 1014, 1016 in their respective open states (step 1110c) and energize the evacuation motor 820 (step 1110d). Accordingly, the insufflation motor 718 drives gas 714 from the gas source 720, through the insufflation module 210, thereby generating insufflation gas 716, through the valve 1004, the positive pressure plenum 1002, the valve 1006, and the insufflation trocar 1008, into the patient's body cavity, while the evacuation motor 820 drives (draws) smoke 812 from the patient's body cavity 1010, through the evacuation trocar 1018, the valve 1016, the negative pressure plenum 1012, the valve 1014, and into the filter 800 of the evacuation module 208, thereby generating filtered air 814.

The controller 620 may then proceed to step 1122 at which the controller 620 may determine if the smoke evacuation is complete. The controller 620 may determine if the smoke evacuation is complete based on receiving a user input, such as at the touchscreen 630, determining if the pressure in the patient's body cavity 1010 (as sensed by sensor 1020) is still at or above the pressure threshold, or any other suitable determination that would necessitate or require the motor 820 of the evacuation module 208 to remain energized, as described elsewhere herein.

If the controller 620 determines that the smoke evacuation is not complete, the controller 620 may loop back to step 1120 and maintain the motors 718, 820 in their activated states. Once the controller 620 determines that the smoke evacuation is complete, the controller 620 may proceed back to step 1108 and determine if the amount of free power of the AC Mains 660 is at or greater than the power threshold to attempt to reprime the positive and negative pressure plenums 1002, 1012.

Accordingly, the foregoing algorithm 1100 enables the controller 620 to pressurize plenums during times at which a threshold amount of power is available from the AC Mains, thereby enabling insufflation gas and smoke to be passively driven to and from the patient's body cavity, respectively, at a time when power from the AC Mains main not be available.

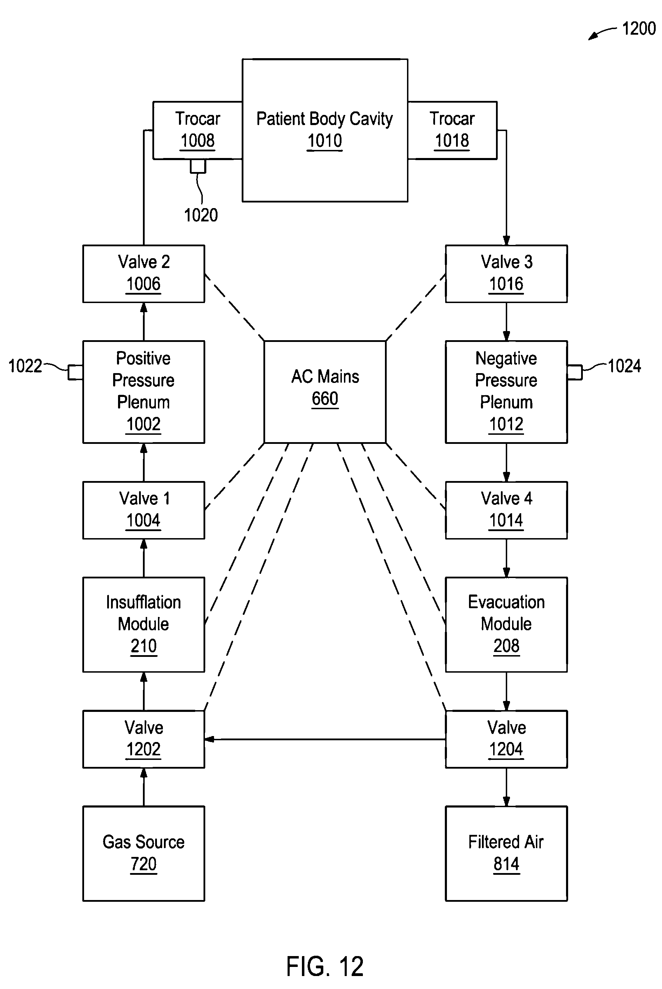

FIG. 12 is a block diagram of another modular surgical system 1200, in accordance with at least one aspect of the present disclosure. The modular surgical system 1200 may be substantially similar to the modular surgical system 1000 of FIG. 10, and therefore may be best understood with reference thereto, where like numerals will correspond to like components not described again in detail. Unlike the modular surgical system 1000, the modular surgical system 1200 includes valves 1202, 1204 (similar to valves 1004, 1006, 1014, 1016 of FIG. 10) that are operable to allow for recirculation of the filtered air 814 to the insufflation module 210.

In some embodiments, valve 1204 may be a three-way valve that is transitionable between a first state, in which the filtered air 814 is exhausted to the operating room (similar to the arrangement in modular surgical system 1000), and a second state, in which the filtered air 814 is passed to the second valve 1202, into the insufflation module 210. The inclusion of valves 1202, 1204 may provide the benefit of using less gas 714 from the gas source 720 during a surgical procedure.

### Three pump system for insufflation and smoke evacuation

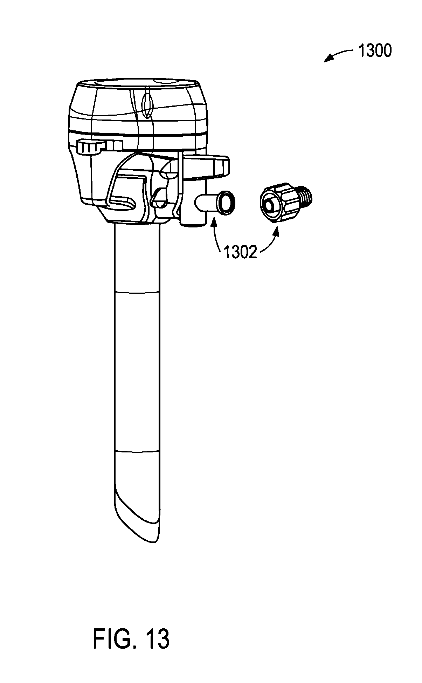

Referring to FIG. 13, a trocar 1300 is illustrated according to at least one aspect of the present disclosure. The trocar 1300 may be similar to the insufflation and evacuation trocars 1008, 1018 of FIG. 10 and may include a luer lock connection 1302 for fluidically coupling to the insufflation or evacuation modules 210, 208. In some instances, the luer lock connection 1302 is the smallest orifice in the system, thereby limiting the flow rate of insufflation gas to a patient's body cavity and/or the flow rate of smoke from the patient's body cavity. One way to increase the flow is to branch the flow into multiple (e.g. two) trocars.

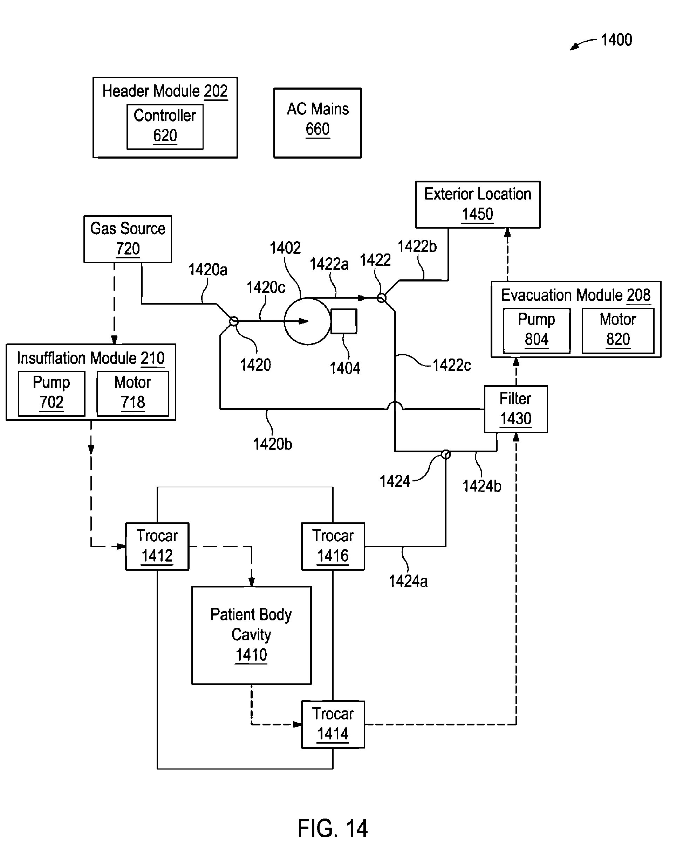

FIG. 14 illustrates a block diagram of a modular surgical system 1400 for increasing the flow rates of insufflation and evacuation, according to at least one aspect of the present disclosure. The modular surgical system 1400 may be similar in arrangement and function to the modular surgical system 600 of FIG. 6. The modular surgical system 1400 may include a header module 202 including a controller 620, an insufflation module 210 including a pump 702 and a motor 718, and an evacuation module 208 including a pump 804 and a motor 820. The modular surgical system 1400 may further include a common pump 1402 and a common motor 1404 for driving the common pump 1402.

The modular surgical system 1400 may further include an AC Mains 660 for providing power to the motors 718, 820, 1404. The controller 620 may be communicably coupled to the motors 718, 820, 1404 (wired or wirelessly), and may be operable to transition each of the motors 718, 820, 1404 between an active state, in which the motors drive the corresponding pump, and an inactive state, in which the motors abstain from driving the corresponding pump. The controller 620 may control various components of the modular surgical system 1400, as will be described in more detail below, such as based on a user providing an input to the controller 620 via the touchscreen 630 (FIG. 6) or based on senor measurements, as described elsewhere herein. The common pump 1402 and motor 1404, as will be described in more detail below, may increase the flow rate of insufflation gas to a patient and the flow rate of smoke out of a patient.

The modular surgical system 1400 may further include an insufflation trocar 1412, an evacuation trocar 1414, and a common trocar 1416, each of which may be similar to the trocar 1300 of FIG. 13, and each of which may be insertable into a patient's body cavity 1410. The insufflation trocar 1412 may be fluidically coupled to the insufflation pump 702, such as via a luer lock connection 1302 (FIG. 13). The trocar 1412 may receive insufflation gas, similar to insufflation gas 716, from the insufflation module 210 for providing insufflation to the patient's body cavity 1410. The evacuation trocar 1414 may be fluidically coupled to the evacuation pump 804 via a secondary filter 1430, similar to filter 800 (FIG. 8) and a luer lock connection 1302 to provide smoke, similar to smoke 812, to the secondary filter 1430.

The modular surgical system 1400 further includes a plurality of three-way valves 1420, 1422, 1424. The first three-way valve 1420 may be transitionable between a first state and a second state. In the first state, the first three-way valve 1420 may fluidically couple a gas source 720 to the common pump 1402 via fluid lines 1420a and 1420c and fluidically decouple the secondary filter 1430 from the common pump 1402. In the second state, the first three-way valve 1420 may fluidically decouple the gas source 720 from the common pump 1402 and fluidically couple the secondary filter 1430 to the common pump 1402 via fluid lines 1420b and 1420c, thereby allowing fluids from the secondary filter 1430 to reach the common pump 1402.

The second three-way valve 1422 may also be transitionable between a first state and a second state. In the first state, the second three-way valve 1422 may fluidically couple the common pump 1402 with an exterior location 1450, such as the operating room, via fluid lines 1422a and 1422b, and fluidically decouple the common pump 1402 from the third three-way valve 1424. In the second state, the second three-way valve 1422 may fluidically decouple the common pump 1402 from the exterior location 1450 and fluidically couple the common pump 1402 to the third three-way valve 1424 via fluid lines 1422a and 1422c.

The third three-way valve 1424 may also be transitionable between a first state and a second state. In the first state, the third three-way valve 1424 may fluidically couple the second three-way valve 1422 (and the common pump 1402) with the common trocar 1416 via fluid lines 1422c and 1424a, and fluidically decouple the common trocar 1416 from the secondary filter 1430. In the second state, the third three-way valve 1424 may fluidically decouple the second three-way valve 1422 (and the common pump 1402) from the common trocar 1416, and fluidically couple the common trocar 1416 with the secondary filter 1430 via fluid lines 1424a and 1424b.

The first, second, and third three-way valves 1420, 1422, 1424 may be any suitable valve that is transitionable (actuatable) between their respective first and second states. In some embodiments, for example, the valves 1420, 1422, 1424 may each include a motor, similar to other motors described elsewhere herein, for transitioning the valves between their respective first and second states. The motors may be powered by a power source, such as the AC Mains 660 or any other suitable power source, and may be communicably coupled to the controller 620 to allow the controller 620 to control the motors. Alternatively, the valves 1420, 1422, 1424 may be solenoid valves which are transitionable between the first and second states by the controller 620 and powered by the power source (e.g. the AC Mains 660).

Still referring to FIG. 14, in operation, the controller 620 may energize the motor 718 to drive (draw) gas from the gas source 720, through the insufflation module 210 (thereby generating insufflation gas 716 (FIG. 7)), the insufflation trocar 1412, and into the patient's body cavity 1410. Similarly, the controller 620 may energize the motor 820 to drive (draw) smoke from the patient's body cavity 1410, through the secondary filter 1430, the evacuation module 208 (thereby generating filtered air 814 (FIG. 8)), and to the exterior location 1450. Depending on the state or configuration of the modular surgical system 1400, the smoke may be drawn from the patient's body cavity 1410 either through the second or third trocars 1414, 1416, as discussed below.

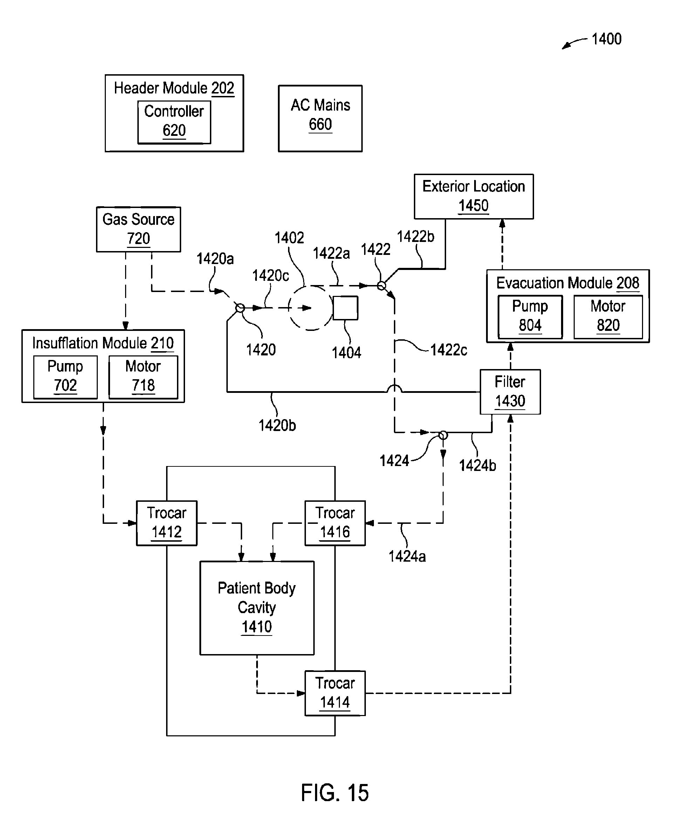

The controller 620 may transition the modular surgical system 1400 between a plurality of states to increase the flow rate of insufflation gas to the patient's body cavity 1410 or increase the flow rate of smoke from the patient's body cavity 1410. Referring now to FIG. 15, the modular surgical system 1400 may be configurable (transitionable) into a first or "insufflation" state, where the controller 620 places the first three-way valve 1420 in the first state, the second three-way valve 1422 in the second state, and the third three-way valve 1424 in the first state. In the insufflation state, the controller 620 energizes the common motor 1404 to operate the common pump 1402 and thereby drive (draw) gas from the gas source 720, through the first three-way valve 1420, the second three-way valve 1422, the third three-way valve 1424, the common trocar 1416, and into the patient's body cavity 1410, thereby providing gas from the gas source 720 to the patient's body cavity 1410 in addition to the insufflation gas provided by the insufflation module 210.

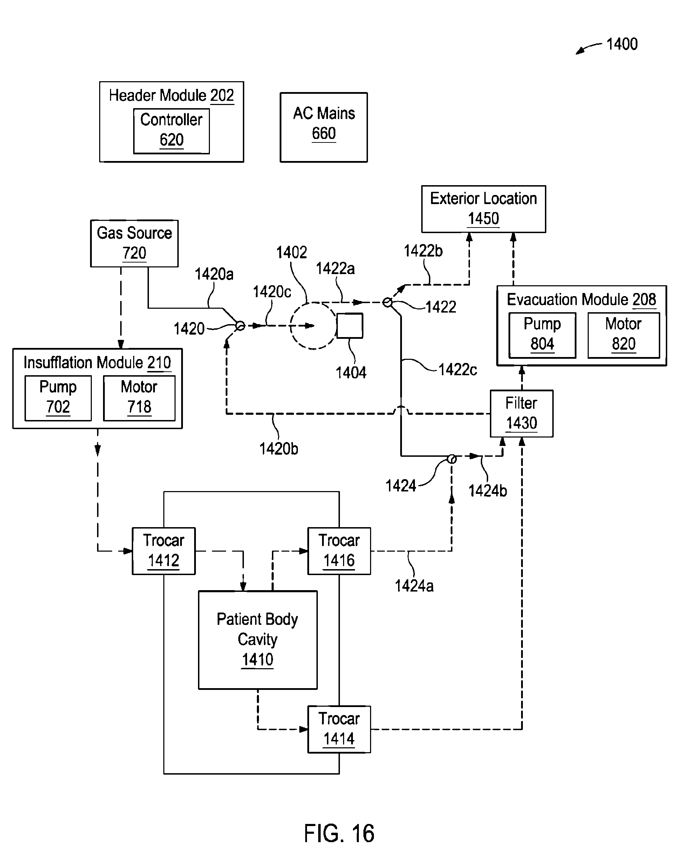

Referring now to FIG. 16, the modular surgical system 1400 may be further configurable (transitionable) into a second or "evacuation" state, where the controller 620 places the first three-way valve 1420 in the second state, the second three-way valve 1422 in the first state, and the third three-way valve 1424 in the second state. In the evacuation state, the controller 620 may energize the motor 1404 to operate the common pump 1402 and thereby drive (draw) smoke from the patient's body cavity 1410 through the common trocar 1416, the third three-way valve 1424, the secondary filter 1430, the first three-way valve 1420, the second three-way valve 1422, and to the exterior location 1450, thereby evacuating smoke from the patient's body cavity 1410 in addition to the smoke that is evacuated by the evacuation module 208.

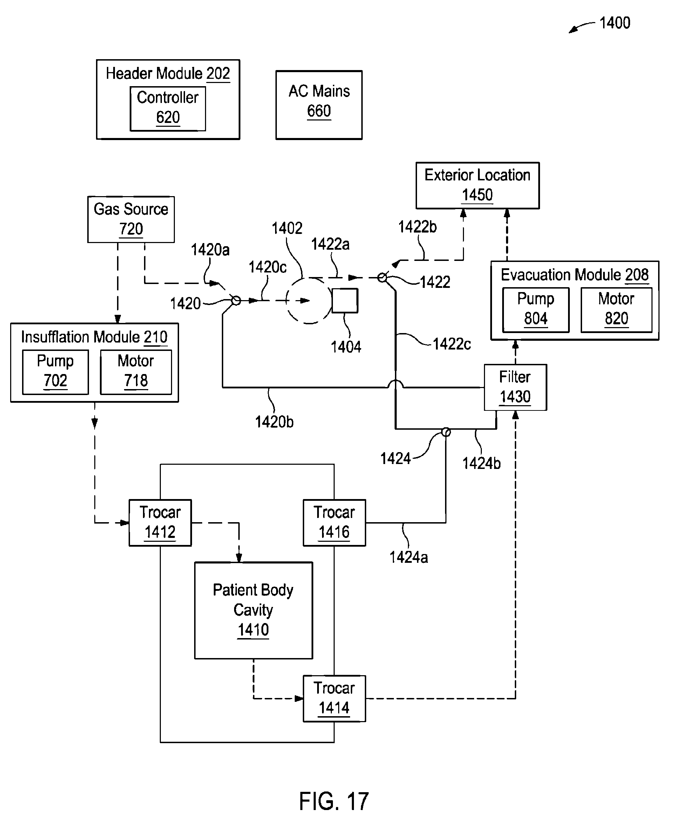

Referring now to FIG. 17, the modular surgical system 1400 may be further configurable into a third or "cleaning" state, where the controller 620 places the first three-way valve 1420 in the first state and the second three-way valve 1422 in the first state. In the cleaning state, the controller 620 may energize the motor 1404 to operate the common pump 1402 and thereby drive (draw) gas from the gas source 720 through the first three-way valve 1420 and the common pump 1402, thereby purging evacuated gas and/or smoke that may still reside in the common pump 1402 out of the common pump 1402 and to the exterior location 1450 via the second three-way valve 1422.

Accordingly, inclusion of the common pump 1402 may enable the modular surgical system 1400 to increase the amount of insufflation gas that can be provided to a patient, and further increase the amount of smoke drawn from the patient than what would typically be capable with a single insufflation module and a single evacuation module. In some embodiments, a humidifier, a heat reservoir, and/or a heater, similar to humidifier 703, heat reservoir 704, and heater 705, respectively, may be placed along line 1420a to heat and humidify the gas that moves from the gas source 720, through the common pump 1402, and to the patient's body cavity 1410, as graphically illustrated in FIG. 15.

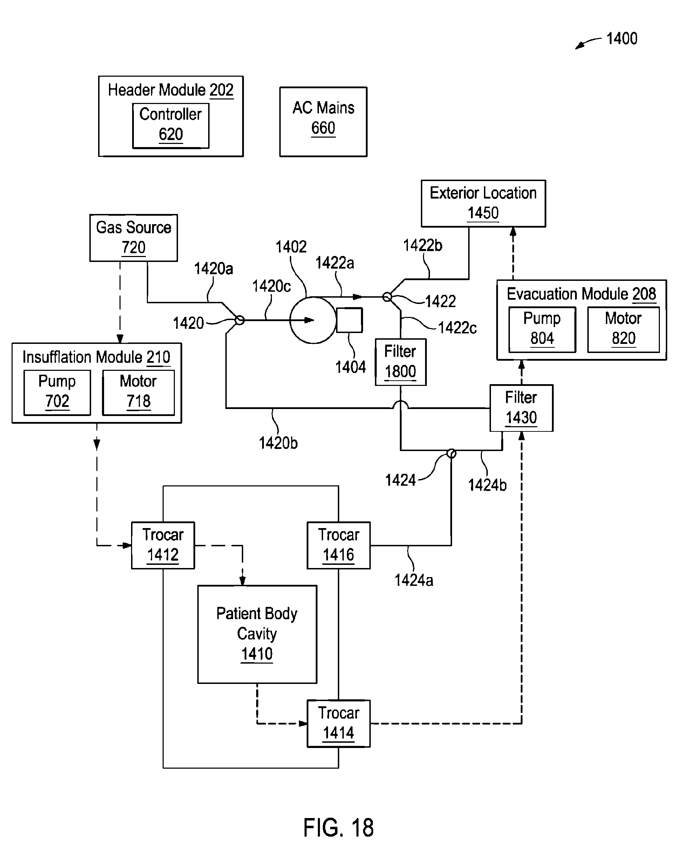

Referring now to FIG. 18, the modular surgical system 1400 may further include a tertiary filter 1800 positioned along line 1422c. The tertiary filter 1800 may filter gas delivered from the common pump 1402. For instance, at a first time, a user may place the modular surgical system 1400 in the evacuation state (FIG. 16), thereby evacuating additional smoke from the patient's body cavity 1410 via the common pump 1402, as described above. Particulate within the smoke may adhere to the common pump 1402 as the smoke moves through the common pump 1402. At a second, later time, the user may place the modular surgical system 1400 in the insufflation state (FIG. 15) to provide additional insufflation to the patient's body cavity 1410, as described above. As gas is delivered to the patient's body cavity 1410, the particulate adhered to the common pump 1402 may be become dislodged from the common pump 1402 and driven towards the patient's body cavity 1410. Accordingly, the tertiary filter 1800 may be arranged and otherwise operable to filter this particulate within the gas.

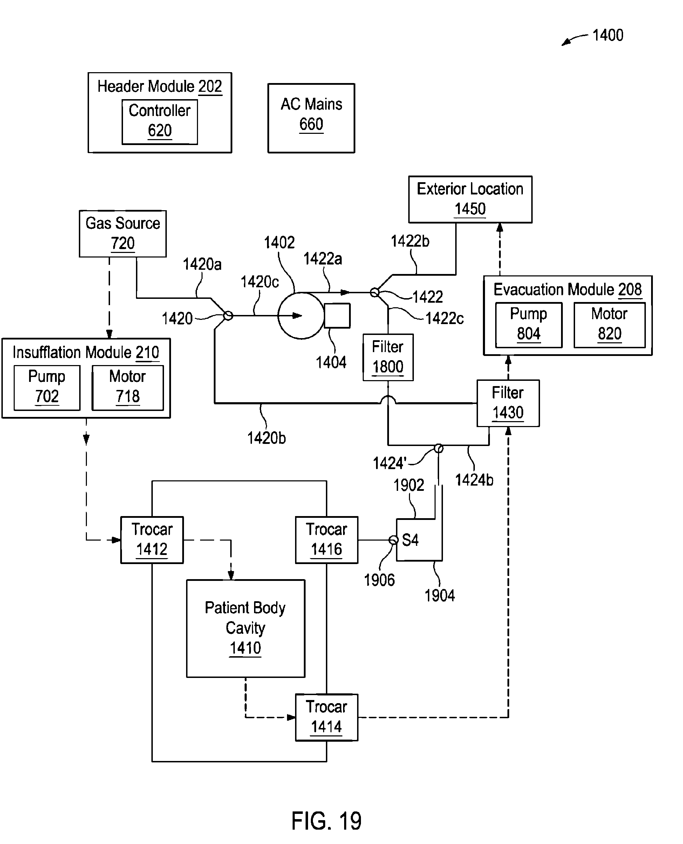

FIG. 19 is a schematic diagram of another modular surgical system 1900 for increasing the flow rates of insufflation and evacuation, according to at least one aspect of the present disclosure. The modular surgical system 1900 may be substantially similar to the modular surgical system 1400 (FIG. 18), except for the differences noted herein below. Accordingly, the modular surgical system 1900 may be best understood with reference to the modular surgical system 1400, where like numerals will correspond to like components not described again in detail.

Notably, unlike modular surgical system 1400 of FIG. 14, the modular surgical system 1900 includes a four-way valve 1424' (in lieu of the third three-way valve 1424 of FIGS. 14-18) and an additional three-way valve 1906. The four-way valve 1424' may be transitionable between a first state and a second state. In the first state, the four-way valve 1424' may fluidically couple the second three-way valve 1422 with the three-way valve 1906 via fluid line 1422c and a first fluid line 1902. In the second state, the four-way valve 1424' may fluidically couple the three-way valve 1906 with the secondary filter 1430 via a second fluid line 1904 and the fluid line 1424b.

The three-way valve 1906 may also be transitionable between a first state and a second state. In the first state, the three-way valve 1906 may fluidically couple the common trocar 1416 to the first fluid line 1902. In the second state, the three-way valve 1906 may fluidically couple the common trocar 1416 to the second fluid line 1904.

Similar to valves 1420, 1422, valves 1424', 1906 may be any suitable valve that is transitionable (actuatable) between their respective first and second states. In some embodiments, the valves 1424', 1906 may each include a motor, similar to the other motors described elsewhere herein, for transitioning the valve 1424', 1906 between their respective first and second states. The motors may be powered by a power source, such as AC Mains 660 or any other suitable power source, and may be communicably coupled to the controller 620 to allow the controller 620 to control the motors. Alternatively, the valves 1424', 1906 may be solenoid valves which are transitionable between the first and second states by the controller 620 and powered by the power source (e.g. AC Mains 660).

Still referring to FIG. 19, in operation, the controller 620 may energize the motor 718 to operate the pump 702 and thereby drive (draw) gas from the gas source 720, through the evacuation module 208 (thereby generating insufflation gas 716 (FIG. 7)), the insufflation trocar 1412, and into the patient's body cavity 1410. Similarly, the controller 620 may energize the motor 820 to operate the pump 804 and thereby drive (draw) smoke from the patient's body cavity 1410, through the secondary filter 1430, the evacuation module 208 (thereby generating filtered air 814), and to the exterior location 1450.

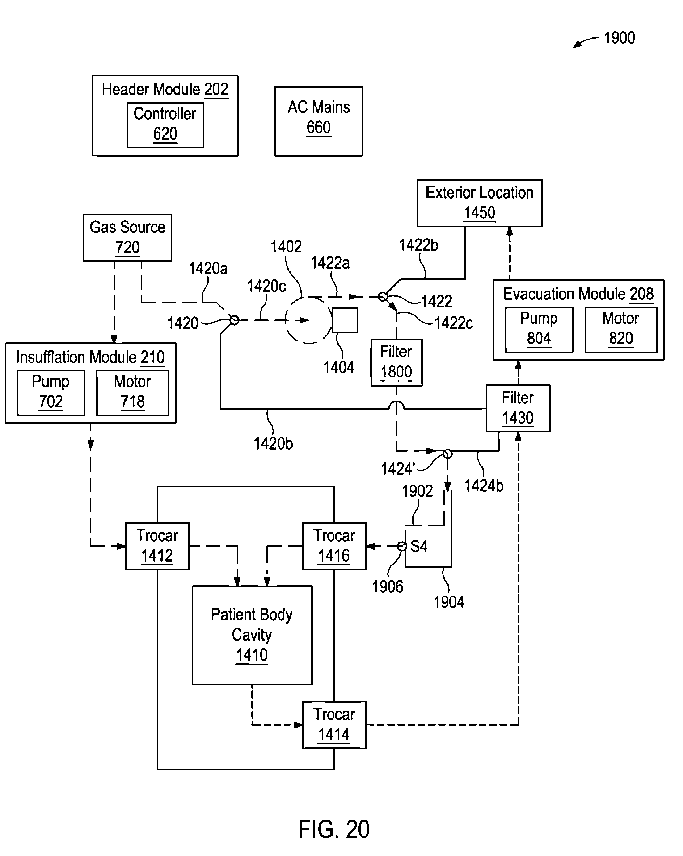

The controller 620 may also transition the modular surgical system 1900 between a plurality of states to increase the flow rate of insufflation gas to the patient's body cavity 1410 or increase the flow rate of smoke from the patient's body cavity 1410. Referring now to FIG. 20, the modular surgical system 1900 may be configurable (transitionable) into a first or "insufflation" state, in which the controller 620 places the first three-way valve 1420 in the first state, the second three-way valve 1422 in the second state, the four-way valve 1424' in the first state, and the three-way valve 1906 in the first state. In the insufflation state, the controller 620 may energize the common motor 1404 to operate the common pump 1402 and thereby drive (draw) gas from the gas source 720, through the first three-way valve 1420, the common pump 1402, the second three-way valve 1422, the tertiary filter 1800, the four-way valve 1424', the first fluid line 1902, the three-way valve 1906, the common trocar 1416, and into the patient's body cavity 1410, thereby providing gas to the patient's body cavity 1410 in addition to the insufflation gas provided by the insufflation module 210.

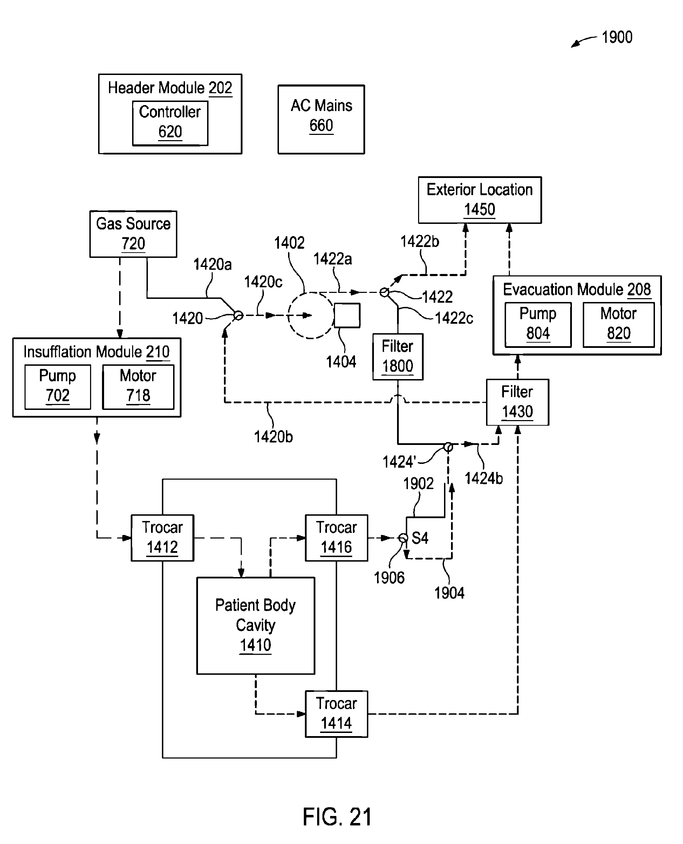

Referring now to FIG. 21, the modular surgical system 1900 may be further configurable into a second or "evacuation" state, in which the controller 620 places the first three-way valve 1420 in the second state, the second three-way valve 1422 in the first state, the four-way valve 1424' in the second state, and the three-way valve 1906 in the second state. In the evacuation state, the controller 620 may energize the common motor 1404 to drive (draw) smoke from the patient's body cavity, through the common trocar 1416, the three-way valve 1906, the second fluid line 1904, the four-way valve 1424', the secondary filter 1430, the first three-way valve 1420, the common pump 1402, the second three-way valve 1422, and to the exterior location 1450, thereby evacuating smoke from the patient's body cavity 1410 in addition to the smoke that is evacuated by (through) the evacuation module 208.

The modular surgical system 1900 may be further configurable into a third or "cleaning" state, similar to the cleaning state of the modular surgical system 1400 (FIG. 14), in which the controller 620 places the first three-way valve 1420 in the first state and the second three-way valve 1422 in the first state. In the cleaning state, the controller 620 may energize the motor 1404 to operate the common pump 1402 and thereby drive (draw) gas from the gas source 720, through the first three-way valve 1420, the common pump 1402, the second three-way valve 1422, and to the exterior location 1450, thereby cleaning the common pump 1402.

Accordingly, inclusion of dedicated fluid lines 1902, 1904, in lieu of a common line, may prevent particulate present in the smoke from becoming entrained within gas that is provided to the patient's body cavity 1410. In some embodiments, a humidifier, a heat reservoir, and/or a heater, similar to humidifier 703, heat reservoir 704, and heater 705, respectively, may be placed along line 1420a to heat and humidify the gas that moves from the gas source 720, through the common pump 1402, and to the patient's body cavity 1410.

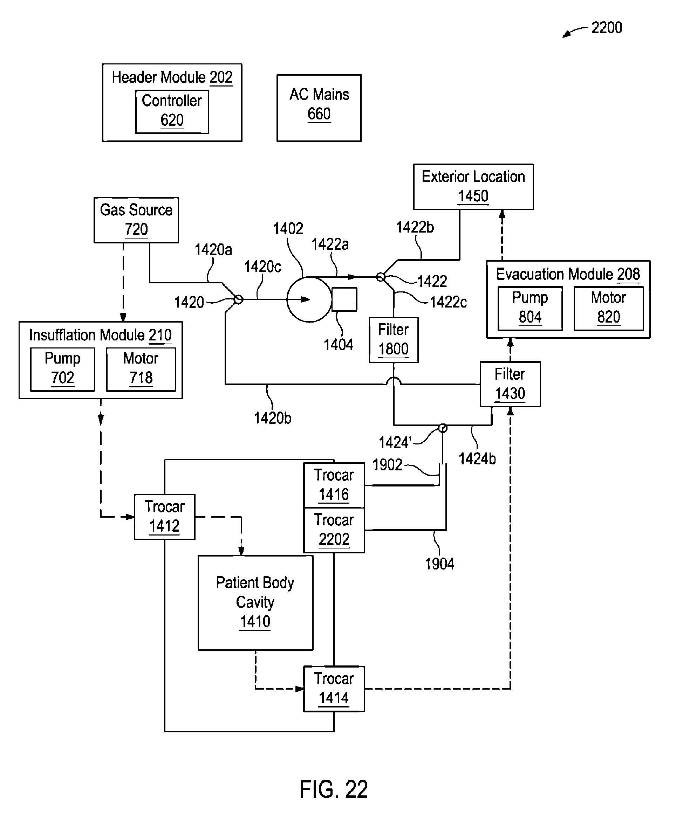

FIG. 22 a schematic diagram of another example modular surgical system 2200 for increasing the flow rates of insufflation and evacuation, according to at least one aspect of the present disclosure. The modular surgical system 2200 may be substantially similar to the modular surgical system 1900 (FIG. 19) except for the differences noted herein below. Notably, like modular surgical system 1900, the modular surgical system 2200 may include the four-way valve 1424', the first fluid line 1902, and the second fluid line 1904. However, unlike modular surgical system 1900, the modular surgical system 2200 omits the three-way valve 1906, and instead includes the common trocar 1416 fluidically coupled to the first fluid line 1902 and a second common trocar 2202 fluidically coupled to the second fluid line 1904. The operation of the modular surgical system 2200 is substantially similar to the modular surgical system 1900, described above, except that, in the first state, the four-way valve 1424' may fluidically couple the second three-way valve 1422 with the common trocar 1416 via fluid lines 1422c, 1902 and, in the second state, the four-way valve 1424' may fluidically couple the secondary filter 1430 with the second common trocar 2202 via the fluid lines 1904, 1424b.

### Automated Closed Loop Pneumostasis System

As discussed elsewhere, an evacuation module 208 (FIG. 2) and an insufflation module 210 (FIG. 2) may be included in a modular surgical system, like modular surgical 600 (FIG. 6), for the purposes of evacuating smoke from a patient and providing insufflation gas to the patient, respectively. A header module 202 (FIG. 2) of the modular surgical system may utilize data provided to and receive from the various modules utilized in the modular surgical system to coordinate and control operation of, among other things, the evacuation module 208 and the insufflation module 210.

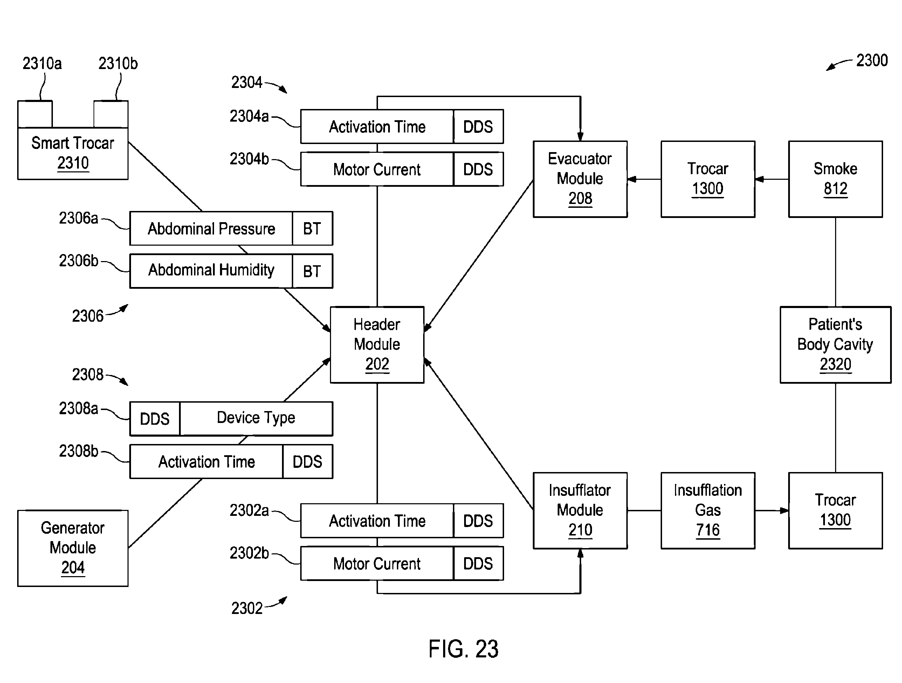

FIG. 23 illustrates a schematic diagram of a modular surgical system 2300, according to at least one aspect of the present disclosure. The modular surgical system 2300 may be similar to modular surgical system 600 (FIG. 6), and therefore may be best understood with reference thereto, where like numerals will correspond to like components not described again. As shown, the header module 202 may provide an activation signal 2302 to the insufflation module 210 that may include an activation time (duration) 2302a and a motor current setting 2302b of the insufflation motor 718 (FIG. 7). The header module 202 may further provide an activation signal 2304 to the evacuation module 208 that may include an activation time (duration) 2304a and a motor current setting 2304b of the evacuation motor 820 (FIG. 8). The activation signals 2302, 2304 may be based on a user providing an input to the touchscreen 630 (FIG. 6) of the controller 620 (FIG. 6) of the header module 202, or based on sensor measurements of the modular surgical system, as discussed elsewhere herein.

The header module 202 may receive a first input signal 2306 from a smart trocar 2310 that is inserted into a patient. The smart trocar 2310 may be similar to trocar 1300 and may include a first sensor 2310a operable to sense abdominal pressure within a patient's body cavity 2320 and a second sensor 2310b operable to sense humidity within the patient's body cavity 2320. The first input signal 2306 includes the abdominal pressure and humidity measurement 2306a, 2306b as measured by the sensors 2310a,b, respectively.

The header module 202 may further receive a second input signal 2308 from a generator module 204. The second input signal 2308 may include a signal indicative of a type of device coupled to the generator module 204, such as one of surgical devices 300, 330, 360 (FIG. 3) and a signal indicative of the amount of time that the device has been activated.

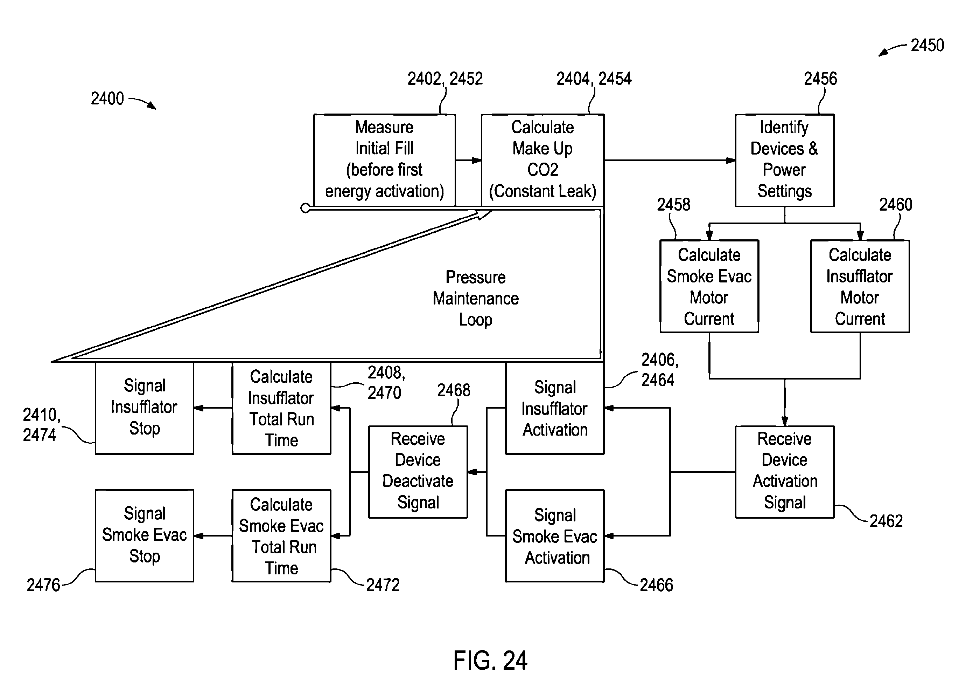

FIG. 24 illustrates flow diagrams 2400, 2450 for controlling the modular surgical system 2300, according to at least one aspect of the present disclosure. In some embodiments, the flow diagrams 2400, 250 are embodied as algorithms, stored in the memory 624 (FIG. 6) of the header module 202 (FIG. 23), and are executable by the processor 622 (FIG. 6) of the header module 202 (FIG. 23). In some embodiments, algorithm 2400 is a pressure maintenance loop algorithm and algorithm 2450 is a coordination algorithm, as will be discussed in more detail below.

Discussion of the flow diagrams 2400, 2500 are now provided with reference to FIG 23. The controller 620 may execute the algorithm 2400, such as in response to a user input at the touchscreen 630 (FIG. 6) or the modular surgical system 2300 being powered on by a power source, such as the AC Mains 660 (FIG. 6). Based on the algorithm 2400 being executed, the controller 620 may provide insufflation gas 716 to the patient's body cavity 2320 via a trocar 1300. At step 2402, the controller 620 may sense the abdominal pressure of the patient's body cavity 2320, such as with the first sensor 2310a of the smart trocar 2310 or with a pressure sensor, like pressure sensor 1020, coupled to the trocar 1300.

The controller 620 may proceed to step 2404 to calculate the leak rate of insufflation in the patient's body cavity 2320. For instance, the controller 620 may monitor the leak rate of the system by monitoring the pressure measurements via first sensor 2310a or pressure sensor on trocar 1300 over time. The controller 620 may proceed to step 2406 to activate (energize) the insufflation module 210 to provide insufflation gas 716 to the patient's body cavity 2320 via trocar 1300. The controller 620 may proceed to step 2406 based on a threshold amount of insufflation pressure being leaked from the patient's body cavity 2320. The threshold amount of insufflation pressure may be stored in the memory 624.

The controller 620 may then proceed to step 2408 to calculate the run time of the insufflation module 210, such as via the timer 626 (FIG. 6). The run time of the insufflation module 210 may be based on the calculated leak rate of the system, calculated at step 2404. The controller 620 may then proceed to step 2410 to stop (de-energize) the insufflation module 210. The controller 620 may proceed to step 2410 based on the measured pressure within the patient's body cavity 2320 reaching a threshold pressure, which may be stored in the memory 624. The controller 620 may then proceed back to step 2404 to calculate the leak rate of insufflation in the patient's body cavity 2320 and repeat the aforementioned pressure maintenance cycle to maintain the insufflation pressure within the patient's body cavity 2320.

The controller 620 may execute the algorithm 2450, such as in response to a user input at the touchscreen 630 (FIG. 6), the modular surgical system 2300 being powered on by a power source, such as the AC Mains 660 (FIG. 6), or a device being coupled to the generator module 204 being activated (energized). Similar to algorithm, based on the algorithm 2450 being executed, the controller 620 may provide insufflation gas 716 to the patient's body cavity 2320 via a trocar 1300. At step 2452, the controller 620 may sense the abdominal pressure of the patient's body cavity 2320, such as with the first sensor 2310a of the smart trocar 2310 or with a pressure sensor, like pressure sensor 1020, coupled to the trocar 1300.

The controller 620 may proceed to step 2454 to calculate the leak rate of insufflation in the patient's body cavity 2320. For instance, the controller 620 may monitor the leak rate of the system by monitoring the pressure measurements via first sensor 2310a or pressure sensor on trocar 1300 over time. The controller 620 may then proceed to step 2456 to identify devices and power settings of the modular surgical system. For instance, the controller 620 may receive an input signal, like input signal 2308, which identifies the device type (ultrasonic device 300, RF device 330, or multifunctional device 360, for example; FIG. 3) and the power settings of the associated device, which may be set at the header module 202.

The controller 620 may then proceed to step 2458 to calculate the motor current of the evacuation motor 820 (FIG. 8) and step 2460 to calculate the motor current of the insufflation motor 718 (FIG. 7). For instance, the controller 620 may measure the current being supplied to the motors 718, 820, such as via current sensors, like other current sensors described elsewhere herein.

The controller 620 may then proceed to step 2462 to receive an activation signal from the device identified at step 2456. For instance, the controller 620 may receive an input signal indicative of the device being activated. Based on the controller 620 receiving the activation signal at step 2462, the controller 620 may proceed to step 2464 to activate (energize) the insufflation module 210 to provide insufflation gas 716 to the patient's body cavity 2320 via trocar 1300 and step 2466 to activate (energize) the evacuation module 208 to draw smoke 812 from the patient's body cavity 2320 via trocar 1300.

The controller 620 may then proceed to step 2468 to receive a deactivation signal of the device. For instance, the controller 620 may receive an input signal indicative of the device being deactivated. Based on the controller 620 receiving the deactivation signal at step 2468, the controller 620 may then proceed to step 2470 to calculate the run time of the insufflation module 210, such as via the timer 626 (FIG. 6) and step 2472 to calculate the run time of the evacuation module 208, such as via the timer 626 (FIG. 6). The run time of the evacuation modular 208 and the insufflation module 210 may be based on the calculated leak rate of the system as calculated at step 2454.

The controller 620 may then proceed to step 2474 to stop (de-energize) the insufflation module 210 and step 2476 to stop (de-energize) the evacuation module 208. The controller 620 may proceed to steps 2474, 2476 based on the measured pressure within the patient's body cavity 2320 reaching a threshold pressure, which may be stored in the memory 624. The controller 620 may then proceed back to step 2454 to calculate the leak rate of insufflation in the patient's body cavity 2320 and repeat the aforementioned cycle to maintain the insufflation pressure within the patient's body cavity 2320.

Embodiments disclosed herein include:
A. A surgical system comprising a power source operable to provide a source amount of power, a first surgical module electrically coupled to the power source, a second surgical module electrically coupled to the power source, and a controller. The first surgical module is transitionable between an idle state in which the first surgical module draws a first amount of power from the power source and an active state in which the first surgical module draws a second amount of power greater than the first amount of power from the power source. The controller is operable to determine a free amount of power available to the second surgical module from the power source. The free amount of power corresponds to a difference between the source amount of power and an amount of power being drawn by the first surgical module.
B. A surgical system comprising a power source operable to provide a source amount of power, a surgical module comprising a pump, a pressure plenum fluidically coupled to the pump and a patient's body cavity, and a first valve, a second valve, and a controller. The first valve is transitionable between an open state to fluidically couple the surgical module and the pressure plenum, and a closed state to fluidically decouple the surgical module from the pressure plenum. The second valve is transitionable between an open state to fluidically couple the pressure plenum and the patient's body cavity and a closed state to fluidically decouple the pressure plenum from the patient's body cavity. The controller is operable to determine a free amount of power available to the motor from the power source, the free amount of power being less than the source amount of power and selectively transition the first and second valves between the open and closed states based on the free amount of power.
C. A surgical system comprising an insufflation pump configured to drive gas into a patient, an evacuation pump configured to draw smoke out of the patient, and a common pump configured to selectively drive gas into the patient and draw smoke out of the patient.

Each of embodiments A-C may have one or more of the following additional elements in any combination: Element 1: wherein the second surgical module comprises an insufflation module. Element 2: wherein the insufflation module comprises a heat reservoir and a heater operable to heat the heat reservoir, wherein the controller is further operable to provide power to the heater up to the determined free amount of power. Element 3: wherein the second surgical module comprises the controller. Element 4: wherein the first surgical module comprises an energy module that provides power to a surgical instrument in the active state and abstains from providing power to the surgical instrument in the idle state. Element 5: further comprising a pressure plenum and first and second valves fluidically coupled to pressure plenum, wherein the first and second valves are each transitionable between an open state to allow gas flow therethrough and a closed state to prevent gas flow therethrough. Element 6: wherein the second surgical module comprises a pump fluidically coupled to the pressure plenum. Element 7: wherein the controller is further operable to compare the free amount of power to a power threshold and based on the free amount of power being greater than the power threshold place the first valve in the open state, place the second valve in the closed state, move gas relative to the pressure plenum with the pump, and place the first valve in the closed state after moving the gas relative to the pressure plenum, thereby generating a pressurized pressure plenum. Element 8: wherein, based on the free amount of power being less than the power threshold, the controller is further operable to place the first and second valves to the open state and move gas through the pressure plenum with the pump. Element 9: wherein the controller is further operable to receive an input and based on receiving the input, place the second valve in the open state to allow passive gas movement between a patient's body cavity and the pressure plenum, thereby depressurizing the pressure plenum. Element 10: wherein the second surgical module comprises an insufflation module and the gas comprises insufflation gas. Element 11: wherein the second surgical module comprises an evacuation module and the gas comprises smoke. Element 12: wherein the controller is further operable to compare the free amount of power to a power threshold and based on the free amount of power being greater the power threshold, place the first valve in the open state, place the second in the closed state, pressurize the pressure plenum with the pump, and place the first valve in the closed state based on the pressure plenum being pressurized, and thereby generating a pressurized pressure plenum. Element 13: wherein the surgical module comprises an insufflation module, and wherein the surgical system further comprises an evacuation module comprising an evacuation pump, a second pressure plenum fluidically coupled to the evacuation pump and the patient's body cavity, a third valve transitionable between an open state to fluidically couple the second pressure plenum and the patient's body cavity and a closed state to fluidically decouple the second pressure plenum from the patient's body cavity, and a fourth valve transitionable between an open state to fluidically couple the evacuation module and the second pressure plenum and a closed state to fluidically decouple the evacuation module from the second pressure plenum. Element 14: further comprising a valve transitionable between a first state in which the valve fluidically couples the common pump with a gas source and a second state in which the valve fluidically couples the common pump with a filter. Element 15: wherein the valve is a first valve and the surgical system further comprises a second valve and a third valve, the second valve being transitionable between a first state, in which the second valve fluidically couples the common pump with an exterior location and a second state, in which the second valve fluidically couples the common pump with the third valve and the third valve being transitionable between a first state in which the third valve fluidically couples the second valve with the patient and a second state in which the third valve fluidically couples the second valve with the filter. Element 16: further comprising an insufflation trocar, an evacuation trocar, and a common trocar, each being insertable into the patient, wherein the insufflation pump is configured to drive gas into the patient via the insufflation trocar, the evacuation pump is configured to draw smoke out of the patient via the evacuation trocar, and the common pump is configured to selectively drive gas into the patient and draw smoke out of the patient via the common trocar. Element 17: further comprising an insufflation trocar, an evacuation trocar, a first common trocar, and a second common trocar, each being insertable into the patient, wherein the insufflation pump is configured to drive gas into the patient via the insufflation trocar, the evacuation pump is configured to draw smoke out of the patient via the evacuation trocar, and the common pump is configured to selectively drive gas into the patient via the first common trocar and draw smoke out of the patient via the second common trocar.

By way of non-limiting example, exemplary combinations applicable to A, B, and C include: Element 1 with Element 2; Element 1 with Element 3; Element 5 with Element 6; Element 5 with Elements 6 and 7; Element 5 with Elements 6-8; Element 5 with Elements 6, 7, and 9; Element 5 with Elements 6, 7, and 10; Element 5 with Elements 6, 7, and 10; Element 5 with Elements 6, 7, and 11; Element 1 with any combination of Elements 2-11; Element 2 with any combination of Elements 1 and 3-11; Element 3 with any combination of Elements 1, 2, and 4-11; Element 4 with any combination of Elements 1-3 and 5-11; Element 5 with any combination of Elements 1-4 and 6-11; Element 6 with any combination of Elements 1-5 and 7-11; Element 7 with any combination of Elements 1-6 and 8-11; Element 8 with any combination of Elements 1-7 and 9-11; Element 9 with any combination of Elements 1-8, 10, and 11; Element 10 with any combination of Elements 1-9 and 11; Element 11 with any combination of Elements 1-10; Element 12 with Element 13; Element 14 with Element 15; Element 14 with Elements 15 and 16; Element 14 with Elements 15 and 17; Element 16 with Element 17; Element 14 with two or more of Elements 15-17.

Therefore, the disclosed systems and methods are well adapted to attain the ends and advantages mentioned as well as those that are inherent therein. The particular embodiments disclosed above are illustrative only, as the teachings of the present disclosure may be modified and practiced in different but equivalent manners apparent to those skilled in the art having the benefit of the teachings herein. Furthermore, no limitations are intended to the details of construction or design herein shown, other than as described in the claims below. It is therefore evident that the particular illustrative embodiments disclosed above may be altered, combined, or modified and all such variations are considered within the scope of the present disclosure. The systems and methods illustratively disclosed herein may suitably be practiced in the absence of any element that is not specifically disclosed herein and/or any optional element disclosed herein. While compositions and methods are described in terms of "comprising," "containing," or "including" various components or steps, the compositions and methods can also "consist essentially of" or "consist of" the various components and steps. All numbers and ranges disclosed above may vary by some amount. Whenever a numerical range with a lower limit and an upper limit is disclosed, any number and any included range falling within the range is specifically disclosed. In particular, every range of values (of the form, "from about a to about b," or, equivalently, "from approximately a to b," or, equivalently, "from approximately a-b") disclosed herein is to be understood to set forth every number and range encompassed within the broader range of values. Also, the terms in the claims have their plain, ordinary meaning unless otherwise explicitly and clearly defined by the patentee. Moreover, the indefinite articles "a" or "an," as used in the claims, are defined herein to mean one or more than one of the elements that it introduces. If there is any conflict in the usages of a word or term in this specification and one or more patent or other documents that may be incorporated herein by reference, the definitions that are consistent with this specification should be adopted.

As used herein, the phrase "at least one of" preceding a series of items, with the terms "and" or "or" to separate any of the items, modifies the list as a whole, rather than each member of the list (i.e., each item). The phrase "at least one of" allows a meaning that includes at least one of any one of the items, and/or at least one of any combination of the items, and/or at least one of each of the items. By way of example, the phrases "at least one of A, B, and C" or "at least one of A, B, or C" each refer to only A, only B, or only C; any combination of A, B, and C; and/or at least one of each of A, B, and C.

The use of directional terms such as above, below, upper, lower, upward, downward, left, right, and the like are used in relation to the illustrative embodiments as they are depicted in the figures, the upward direction being toward the top of the corresponding figure and the downward direction being toward the bottom of the corresponding figure.

## Claims

1. A surgical system, comprising:
a power source operable to provide a source amount of power;
a first surgical module electrically coupled to the power source and transitionable between:
an idle state, in which the first surgical module draws a first amount of power from the power source; and
an active state, in which the first surgical module draws a second amount of power greater than the first amount of power from the power source;
a second surgical module electrically coupled to the power source; and
a controller operable to determine a free amount of power available to the second surgical module from the power source,
wherein the free amount of power corresponds to a difference between the source amount of power and an amount of power being drawn by the first surgical module.

2. The surgical system of Claim 1, wherein the second surgical module comprises
an insufflation module;
optionally wherein the insufflation module comprises:
a heat reservoir; and
a heater operable to heat the heat reservoir,
wherein the controller is further operable to provide power to the heater up to the determined free amount of power.

3. The surgical system of Claim 1, wherein the second surgical module comprises the controller.

4. The surgical system of any one of Claims 1 to 3, wherein the first surgical module comprises an energy module that provides power to a surgical instrument in the active state and abstains from providing power to the surgical instrument in the idle state.

5. The surgical system of any one of Claims 1 to 4, further comprising:
a pressure plenum; and
first and second valves fluidically coupled to pressure plenum, wherein the first and second valves are each transitionable between:
an open state to allow gas flow therethrough; and
a closed state to prevent gas flow therethrough.

6. The surgical system of Claim 5, wherein the second surgical module comprises a pump fluidically coupled to the pressure plenum.

7. The surgical system of Claim 6, wherein the controller is further operable to:
compare the free amount of power to a power threshold; and
based on the free amount of power being greater than the power threshold:
place the first valve in the open state;
place the second valve in the closed state;
move gas relative to the pressure plenum with the pump; and
place the first valve in the closed state after moving the gas relative to the pressure plenum, thereby generating a pressurized pressure plenum; optionally wherein, based on the free amount of power being less than the power threshold, the controller is further operable to:
place the first and second valves to the open state; and
move gas through the pressure plenum with the pump.

8. The surgical system of Claim 7, wherein the controller is further operable to:
receive an input; and
based on receiving the input, place the second valve in the open state to allow passive gas movement between a patient's body cavity and the pressure plenum, thereby depressurizing the pressure plenum.

9. The surgical system of Claim 7, wherein either:
the second surgical module comprises an insufflation module and the gas comprises insufflation gas; or
the second surgical module comprises an evacuation module and the gas comprises smoke.

10. The surgical system of Claim 6, wherein:
the pressure plenum is fluidically coupled to a patient's body cavity;
the open state of the first valve fluidically couples the surgical module and the pressure plenum, and the closed state of the first valve fluidically decouples the surgical module from the pressure plenum;
the open state of the second valve fluidically couples the pressure plenum and the patient's body cavity, and the closed state of the second valve fluidically decouples the pressure plenum from the patient's body cavity; and
the controller is operable to selectively transition the first and second valves between the open and closed states based on the free amount of power.

11. The surgical system of Claim 10, wherein the surgical module comprises an insufflation module, and wherein the surgical system further comprises:
an evacuation module comprising an evacuation pump;
a second pressure plenum fluidically coupled to the evacuation pump and the patient's body cavity;
a third valve transitionable between an open state to fluidically couple the second pressure plenum and the patient's body cavity and a closed state to fluidically decouple the second pressure plenum from the patient's body cavity; and
a fourth valve transitionable between an open state to fluidically couple the evacuation module and the second pressure plenum and a closed state to fluidically decouple the evacuation module from the second pressure plenum.

12. A surgical system, comprising:
an insufflation pump configured to drive gas into a patient;
an evacuation pump configured to draw smoke out of the patient; and
a common pump configured to selectively drive gas into the patient and draw smoke out of the patient.

13. The surgical system of Claim 12, further comprising a valve transitionable between:
a first state in which the valve fluidically couples the common pump with a gas source; and
a second state in which the valve fluidically couples the common pump with a filter;
optionally wherein the valve is a first valve and the surgical system further comprises:
a second valve and a third valve, the second valve being transitionable between:
a first state, in which the second valve fluidically couples the common pump with an exterior location; and
a second state, in which the second valve fluidically couples the common pump with the third valve; and
the third valve being transitionable between:
a first state in which the third valve fluidically couples the second valve with the patient; and
a second state in which the third valve fluidically couples the second valve with the filter.

14. The surgical system of Claim 12, further comprising an insufflation trocar, an evacuation trocar, and a common trocar, each being insertable into the patient, wherein:
the insufflation pump is configured to drive gas into the patient via the insufflation trocar;
the evacuation pump is configured to draw smoke out of the patient via the evacuation trocar; and
the common pump is configured to selectively drive gas into the patient and draw smoke out of the patient via the common trocar.

15. The surgical system of Claim 12, further comprising an insufflation trocar, an evacuation trocar, a first common trocar, and a second common trocar, each being insertable into the patient, wherein:
the insufflation pump is configured to drive gas into the patient via the insufflation trocar;
the evacuation pump is configured to draw smoke out of the patient via the evacuation trocar; and
the common pump is configured to selectively drive gas into the patient via the first common trocar and draw smoke out of the patient via the second common trocar.
